# EUROPÄISCHE PATENTSCHRIFT

(11) **EP 1 325 154 B1**
(45) Veröffentlichungstag und Bekanntmachung des Hinweises auf die Patenterteilung: **01.08.2007**
(21) Anmeldenummer: 01986724.1
(22) Anmeldetag: 10.10.2001
(51) Int. Cl.: C12Q 1/68

(54) **VERFAHREN ZUM NACHWEIS VON CYTOSIN-METHYLIERUNGEN**
METHOD FOR THE DETECTION OF CYTOSINE METHYLATIONS
PROCEDE DE DETECTION DE LA METHYLATION DE LA CYTOSINE

(30) Priorität: 10.10.2000 DE 10050942
(43) Veröffentlichungstag der Anmeldung: 09.07.2003
(73) Patentinhaber: Epigenomics AG, 10178 Berlin (DE)
(72) Erfinder: BERLIN, Kurt, 14532 Stahnsdorf (DE)
(74) Vertreter: Schubert, Klemens
(86) Internationale Anmeldenummer: PCT/DE2001/003901
(87) Internationale Veröffentlichungsnummer: WO 2002/031186

(56) Entgegenhaltungen:
- WO-A-99/28498
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048
- PAULIN RICHARD ET AL: "Urea improves efficiency of bisulphite-mediated sequencing of 5'-methylcytosine in genomic DNA" NUCLEIC ACIDS RESEARCH, OXFORD UNIVERSITY PRESS, SURREY, GB, Bd. 26, Nr. 21, 1. November 1998 (1998-11-01), Seiten 5009-5010, XP002210104 ISSN: 0305-1048

## Beschreibung

Die vorliegende Erfindung beschreibt ein Verfahren zur Detektion des Methylierungszustandes genomischer DNA Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek A, Oswald J, Walter J. A modified and improved method for bisulphite based cytosine methylation analysis. Nucleic Acids Res. 1996 Dec 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden:Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifziert und entweder komplett sequenziert (Olek A, Walter J. The pre-implantation ontogeny of the H19 methylation imprint. Nat Genet. 1997 Nov;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun 15;25(12):2532-4) nachgewiesen. Zudem ist auch der Nachweis durch Hybridisierung beschrieben worden (Olek et al., WO 99 28498).

Ein Verfahren zur Analyse von Cytosinmethylierungen mittels der Herstellung komplexer DNA-Methylierungs-Fingerabdrücke ist in der WO 99/28498 beschrieben. Dabei wird die zu analysierende DNA zunächst mit Bisulfit umgewandelt und anschließend mit Hilfe sehr kurzer oder degenerierter Primer amplifiziert.

Weitere Publikationen, die sich mit der Anwendung der Bisulfit-Technik zum Methylierungsnachweis bei einzelnen Genen befassen, sind:
Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nu-cleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97 46705, WO 95 15373 und WO 95 45560.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonukleotide bei vermindertem nichtspezifischem Hintergrundsignal entnehmen.

Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags*"* an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis, Molecular Cloning: A Laboratory Manual, 1989.

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphite-mediated sequencing of 5'-methylcytosine in genomic DNA. Nucleic Acids Res. 1998 Nov 1;26(21):5009-10).

Es gibt demnach Verfahren, die es erlauben, Cytosin-Methylierung dadurch zu identifizieren, dass alle nicht methylierten Cytosine in eine bestimmte Base umgewandelt werden und lediglich die methylierten Cytosine nach der Amplifikation noch als Cytosin vorliegen. Damit ist es jedoch nicht ohne weiteres möglich, umgekehrt nach der Amplifikation alle noch vorhandenen Cytosinbasen ehemals methylierten Cytosinen zuzuordnen, da bei der Amplifikation im Gegenstrang immer noch Cytosin als Komplementäres zu dem nicht umgewandelten Guanin eingebaut wird. Dies stört jedoch, will man in einem einfachen Verfahren für Cytosin charakteristische Reaktionen ausnutzen, um methylierte Positionen zu bestimmen oder das Ausmaß der Methylierung in dem amplifizierten Abschnitt festzustellen. Dieses Problem soll in der vorliegenden Erfindung gelöst werden.

Es ist Aufgabe der Erfindung, ein Verfahren bereitzustellen, das es erlaubt, in eine amplifizierte doppelsträngige DNA Markierungen einzubauen, die zur eindeutigen Identifizierung von Cytosin-Methylierungen in genomischen DNA-Proben genutzt werden können. Diese Markierungen sollen die Verwendung einfacher etablierter molekularbiologischer Methoden zur Methylierungsanalyse erlauben.

Die Aufgabe wird erfindungsgemäß durch ein Verfahren zur Detektion von Cytosin-Methylierung in genomischer DNA gelöst, wobei man die folgenden Verfahrensschritte ausführt:
a) man setzt eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird;
b) man amplifiziert die vorbehandelte DNA unter Verwendung einer Polymerase und mindestens eines Primeroligonukleotids;
c) man setzt das Amplifikat erneut mit einem Reagenz chemisch um, wobei 5-Methylcytosin unverändert bleibt und Cytosin in Uracil oder eine andere im Basenpaarungsverhalten dem Uracil ähnliche Base umgewandelt wird;
d) man weist die in dem Amplifikat nach der erneuten chemischen Behandlung verbleibenden Cytosinbasen und/oder Guaninbasen nach.

Die Aufgabe wird ferner durch ein Verfahren zur Detektion von Cytosin-Methylierung in genomischer DNA gelöst, wobei man die folgenden Verfahrensschritte ausführt:
a) man setzt eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird;
b) man amplifiziert die vorbehandelte DNA unter Verwendung einer Polymerase und mindestens eines Primeroligonukleotids;
c) man setzt das Amplifikat erneut mit einem Reagenz chemisch um, wobei 5-Methylcytosin unverändert bleibt und Cytosin in Uracil oder eine andere im Basenpaarungsverhalten dem Uracil ähnliche Base umgewandelt wird;
d) man amplifiziert die chemisch behandelten Amplifikate aus Schritt c) erneut;
e) man weist die in dem Amplifikat verbleibenden Cytosinbasen und/oder Guaninbasen nach. Diese erfindungsgemäße Verfahrenvariante umfasst zwei Amplifizierungsschritte.

Besonders bevorzugt ist bei den erfindungsgemäßen Verfahren dass die chemische Behandlung mit Natriumbisulfit (=Hydrogensulfit, Disulfit) erfolgt. Dabei ist es ferner besonders bevorzugt, dass die chemische Behandlung nach Einbetten der DNA in Agarose erfolgt.

Beim erfindungsgemäßen Verfahren ist bevorzugt, dass bei der chemischen Behandlung ein die DNA-Duplex denaturierendes Reagenz und/oder ein Radikalfänger zugegen ist. Besonders bevorzugt ist dabei, dass das denaturierende Reagenz aus einer der folgenden Substanzen ausgewählt ist:
Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF und/oder dass der Radikalfänger aus einer der folgenden Substanzen ausgewählt ist:
Di-, Trihydroxybenzole, green tea extract, pine bark extract (Pycnogenol), Ginkgo Biloba extract (EGb 761), a flavonoid blend of several fruit and vegetable extracts (GNLD), Bio-Normalizer (Sun-O Corp), DPPH (1,1-Diphenyl-2-picrylhydrazyl), NDGA (Nordihydroguajaret-säure), Trolox (6-Hydroxy-2, 5, 7, 8-tetramethylchroman-2-carboxylic acid), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, E, Q, Hydrochinon, Ubichinon, Lignane, Hydroxyterpene, Flavonoide, Curcumin, Tannine, Retinsäureverbindungen, Ge-132 Bisbetacarboxyethyl germanium sesquioxide, Superoxid dismutase (SOD), Superoxid katalase, Alpha-Naphthoflavone, Ginkgo biloba extract (EGb 761), Di(2-methyl-5-chlorophenyl)dithionate und Cu(II)-Derivate, Mebendazole, CS (Chloroform soluble) alkaloidal extract, 4-(3,5-Di-tert-butyl-4-hydroxyphenyl)-3-hydroxy-1,2-naphthochinon, 4- (3, 5-Di-tert-butyl-4-hydroxyphenyl) -3-methoxy-1, 2-naphthochinon, 4-(3, 5-Di-tert-butyl-4-hydroxyphenyl)-1,2-naphthochinon, 2-(3, 5-Di-tert-butyl-4-hydroxyphenyl)-3-brom-1,4-naphthochinon, 2-(3, 5-Di-tert-butyl-4-hydroxyphenyl) -3-chlor-1, 4-naphthochinon, 2-(3, 5-Di-tert-butyl-4-hydroxyphenyl)-3-methoxy-1, 4-naphthochinon, 2-(3,5-Di-tert-butyl-4-hydroxyphenyl)-3-hydroxy-1, 4-naphthochinon, 2-(3, 5-Di-tert-butyl-4-hydroxyphenyl)-1, 4-naphthochinon, 4-(3,5-Di-tert-butyl-4-hydroxyphenyl)-3-hydroxy-5,5,8,8-tetramethyl-5,6,7,8-tetrahydro-1,2-anthrachinon, 4-(3,5-Di-tert-butyl-4-hydroxyphenyl)-3-methoxy-5,5,8,8-tetramethyl-5,6,7,8-tetrahydro-1,2-anthrachinon, 4-(3,5-Di-tert-butyl-4-hydroxyphenyl)-5,5,8,8-tetramethyl-5,6,7,8-tetrahydro-1,2-anthrachinon, 3-Brom-4-(3,5-di-tert-butyl-4-hydroxyphenyl)-5,5,8,8-tetramethyl-5,6,7,8-tetrahydro-1,2-anthrachinon, 2-(3,5-Di-tert-butyl-4-oxocyclohexa-2,5-dienyliden)-indan-1,3-dion, 2-(3,5-Di-tert-butyl-4-oxocyclohexa-2,5-dienyliden)-3,4-epoxy-3-hydroxy-4-methoxy-3,4-dihydro-2H-naphthalin-1-on, 2-(3,5-Di-tert-butyl-4-oxocyclohexa-2,5-dienyliden)-3,4-epoxy-3,4-dimethoxy-3,4-dihydro-2H-naphthalin-1-on, 2-(3,5-Di-tert-butyl-4-hydroxyphenyl)-indan-1-on, 3,3-Bi-[2-(3,5-di-tert-butyl-4-hydroxyphenyl)-inden-1-on]-3-yl, 2-(3,5-Di-tert-butyl-4-hydroxyphenyl)-3-brom-5,5,8,8-tetramethyl-5,6,7,8-tetrahydro-1,4-anthrachinon, 2-(3,5-Di-tert-butyl-4-hydroxyphenyl)-3-chlor-5,5,8,8-tetramethyl-5,6,7,8-tetrahydro-1,4-anthrachinon, 2-(3,5-Di-tert-butyl-4-hydroxyphenyl)-3-methoxy-5,5,8,8-tetramethyl-5,6,7,8-tetrahydro-1,4-anthrachinon, 2-(3,5-Di-tert-butyl-4-hydroxyphenyl)-3-hydroxy-5,5,8,8-tetramethyl-5,6,7,8-tetrahydro-1,4-anthrachinon, 2-(3,5-Di-tert-butyl-4-hydroxyphenyl)-5,5,8,8-tetramethyl-5,6,7,8-tetrahydro-1,4-anthrachinon, 2-Brom-3- (3-brom-5-tert-butyl-4-hydroxyphenyl)-5,5,8,8-tetramethyl-5,6,7,8-tetrahydro-1,4-anthrachinon, 2-Brom-3-(3,5-dibrom-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-3-hydroxy-5,5,8,8-tetramethyl-5,6,7,8-tetrahydro-1,4-anthrachinon, 3-Brom-2- (3, 5-di-tert-butyl-4-hydroxyphenyl)-1,4-anthrachinon, 2-(3, 5-Di-tert-butyl-4-hydroxyphenyl)-3-methoxy-1,4-anthrachinon, 2-(3, 5-Di-tert-butyl-4-hydroxyphenyl)-3-hydroxy-1,4-anthrachinon, 5,5,8,8-Tetramethyl-5,6,7,8-tetrahydronaphthalin-1,3-diol, 3-Methoxy-5,5,8,8-tetramethyl-5,6,7,8-tetrahydronaphthalin-1-ol, 4-(3-Chlor-5,5,8,8-tetramethyl-1,4-dioxo-1,4,5,6,7,8-hexahydroanthracen-2-yl)-benzoesäure, Methyl-4-(3-chlor-5,5,8,8-tetramethyl-1,4-dioxo-1,4,5,6,7,8-hexahydroanthracen-2-yl)-benzoat, 4- (3-Hydroxy-1,4-dioxo-1,4-dihydronaphthalin-2-yl)-benzoesäure, Methyl-(3-methoxy-1,4-dioxo-1,4-dihydronaphthalin-2-yl)-benzoesäure, 4- (3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat, 4-(3-Hydroxy-5,5,8,8-tetramethyl-1,4-dioxo-1,4,5,6,7,8-hexahydroanthracen-2-yl-azo)-benzoesäure, 3-(3,5-Di-tert-butyl-4-oxocyclohexa-2,5-dienyliden)-5,5,8,8-tetramethyl-5,6,7,8-tetrahydrocyclopenta[b]naphthalin-1,2-dion, 3-(3,5-Di-tert-butyl-4-oxocyclohexa-2,5-dienyliden)-5,5,8,8-tetramethyl-5,6,7,8-tetrahydroanthracen-3H-1,2,4-trion, 2-(3,5-Di-tert-butyl-4-hydroxyphenyl)-3-methoxy-5,8-dimethyl-1,4-naphthochinon, 2-(3,5-Di-tert-butyl-4-hydroxyphenyl)-3-methoxy-6,7-dimethyl-1,4-naphthochinon, 2-(3,5-Di-tert-butyl-4-hydroxyphenyl)-3-methoxy-5-methyl-1, 4-naphthochinon, 2-(3,5-Di-tert-butyl-4-hydroxyphenyl)-2-methoxy-5-methyl-1,4-naphthochinon, 2-(3, 5-Di-tert-butyl-4-hydroxyphenyl)-3-methoxy-6-methyl-1,4-naphthochinon, 3-(3,5-Di-tert-butyl-4-hydroxyphenyl)-2-methoxy-6-methyl-1,4-naphthochinon, 2-(3,5-Di-tert-butyl-4-hydroxyphenyl)-3-methoxy-5, 6-dimethyl-1, 4-naphthochinon, 3-(3,5-Di-tert-butyl-4-hydroxyphenyl)-2-methoxy-5,6-dimethyl-1,4-naphthochinon, 2-(3,5-Di-tert-butyl-4-hydroxyphenyl)-3-methoxy-5,7-dimethyl-1,4-naphthochinon, 3-(3,5-Di-tert-butyl-4-hydroxyphenyl)-2-methoxy-5,7-dimethyl-1,4-naphthochinon, 2-(3,5-Di-tert-butyl-4-hydroxyphenyl)-3-ethylthio-5-methyl-1,4-naphthochinon, 2-(3, 5-Di-tert-butyl-4-hydroxyphenyl)-3-ethylthio-6-methyl-1,4-naphthochinon, 2-(3,5-Di-tert-butyl-4-hydroxyphenyl)-3-hydroxy-5,8-dimethyl-1,4-naphthochinon, 2-(3, 5-Di-tert-butyl-4-hydroxyphenyl)-3-hydroxy-6,7-dimethyl-1,4-naphthochinon, 2-(3,5-Di-tert-butyl-4-hydroxyphenyl)-3-hydroxy-5-methyl-1, 4-naphthochinon, 3-(3,5-Di-tert-butyl-4-hydroxyphenyl)-2-hydroxy-5-methyl-1,4-naphthochinon, 2-(3,5-Di-tert-butyl-4-hydroxyphenyl)-3-hydroxy-6-methyl-1,4-naphthochinon, 3-(3,5-Di-tert-butyl-4-hydroxyphenyl)-2-hydroxy-6-methyl-1, 4-naphthochinon, 2-(3,5-Di-tert-butyl-4-hydroxyphenyl) -3-hydroxy-5, 6-dimethyl-1, 4-naphthochinon, 2-(3-Brom-5-tert-butyl-4-hydroxyphenyl)-3-hydroxy-5,6-dimethyl-1,4-naphthochinon, 3- (3, 5-Di-tert-butyl-4-hydroxyphenyl)-2-hydroxy-5,6-dimethyl-1,4-naphthochinon, 2-(3,5-Di-tert-butyl-4-hydroxyphenyl)-3-hydroxy-5,7-dimethyl-1,4-naphthochinon, 3-(3,5-Di-tert-butyl-4-hydroxyphenyl)-2-hydroxy-5,7-dimethyl-1,4-naphthochinon.

Erfindungsgemäß ist auch bevorzugt, dass man Reagenzien, die für die chemische Behandlung der DNA eingesetzt werden, vor der jeweils nachfolgenden Amplifikation ganz oder teilweise abtrennt. Bevorzugt ist auch, dass man die Probe nach der chemischen Behandlung vor der Amplifikation verdünnt. Weiterhin ist erfindungsgemäß bevorzugt, dass man die Amplifikation von mehreren DNA-Abschnitten gleichzeitig in einem Reaktionsgefäß durchführt und/oder dass man für die Amplifikation eine hitzebeständige DNA-Polymerase verwendet.

Ganz besonders bevorzugt ist aber auch ein erfindungsgemäßes Verfahren, wobei man vor den Amplifikationen eine Desulfonierung der DNA durchführt.

Bevorzugt ist auch, dass man die nach der zweiten chemischen Behandlung verbleibenden Cytosin- und/oder Guaninbasen durch Hybridisierungsreaktionen nachweist und/oder dass man die nach der zweiten chemischen Behandlung verbleibenden Cytosin- und/oder Guaninbasen durch spezifischen Einbau von nachweisbaren Markierungen an den Cytosin- und/oder Guaninbasen nachweist und/oder quantifiziert.

Bevorzugt ist erfindungsgemäß auch ein Verfahren, wobei man in Schritt d) gemäß der ersten Verfahrensvariante oder Schritt e) gemäß der zweiten Verfahrensvariante für die Detektion der vorbehandelten DNA die Amplifikationsprodukte an einen Oligonukleotid Array hybridisiert und man anschliessend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man verlängert das Oligonukleotid mit bekannter Sequenz von n Nukleotiden mittels einer Polymerase mindestens um ein Nukleotid, wobei das Nukleotid eine nachweisbare Markierung trägt und die Verlängerung vom Methylierungsstatus des jeweiligen Cytosins in der genomischen DNA-Probe abhängt.

Bevorzugt ist dabei auch, dass man die nach der zweiten chemischen Behandlung verbleibenden Cytosin- und/oder Guaninbasen durch Sequenzierungsreaktionen nachweist.

Weiterhin ist bevorzugt, dass man in Schritt d) gemäß der ersten Verfahrenvariante oder Schritt e) gemäß der zweiten Verfahrensvariante für die Detektion der vorbehandelten DNA die Amplifikationsprodukte an einen Oligonukleotid Array hybridisiert und man anschliessend die folgenden Teilschritte ausführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) man verlängert das Oligonukleotid der ersten Spezies mit bekannter Sequenz von n Nukleotiden mittels einer Polymerase um höchstens die Anzahl von Nukleotiden, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

Dabei ist weiterhin besonders bevorzugt, dass man für die Detektion der vorbehandelten DNA gemäß Schritt d) in der ersten Verfahrensvariante die PCR-Produkte auf einen Oligonukleotid Array hybridisiert und man anschliessend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man verlängert das Oligonukleotid, sofern es mit seinem 3'Terminus zuvor ohne Basenfehlpaarungen an die zu untersuchenden Position hybridisierte, mittels einer Polymerase mindestens um ein Nukleotid, wobei mindestens ein Nukleotid eine nachweisbare Markierung trägt und die Verlängerung vom Methylierungsstatus des jeweiligen Cytosins in der genomischen DNA-Probe abhängt.

Bevorzugt ist auch ein erfindungsgemäßes Verfahren, wobei man die PCR-Produkte und/oder Verlängerungsprodukte und/oder Ligationsprodukte für die Detektion mit einer nachweisbaren Markierung versieht. Bevorzugt ist hierbei, dass die Markierungen Fluoreszenzmarkierungen sind oder dass die Markierungen Radionuklide sind oder dass die Markierungen ablösbare Massenmarkierungen sind, die in einem Massenspektrometer nachgewiesen werden.

Bevorzugt ist auch, dass bei einer der Amplifikationen einer der Primer an eine Festphase gebunden ist.

Insbeondere ist bevorzugt, dass man die PCR-Produkte und/oder Verlängerungsprodukte und/oder Ligationsprodukte insgesamt im Massenspektrometer nachweist und somit durch ihre Masse eindeutig charakterisiert sind. Bevorzugt ist auch, dass man jeweils ein Fragment der PCR-Produkte und/oder Verlängerungsprodukte und/oder Ligationsprodukte im Massenspektrometer nachweist. Hierbei ist besonders bevorzugt, dass man das Fragment des PCR-Produkts und/oder Verlängerungsprodukts und/oder Ligationsprodukts durch Verdau mit einer oder mehrerer Exo- oder Endonukleasen erzeugt. Ganz besonders bevorzugt ist hierbei, dass zur besseren Detektierbarkeit im Massenspektrometer die erzeugten Fragmente eine einzelne positive oder negative Nettoladung aufweisen.

Bevorzugt ist erfindungsgemäß auch dass man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

Besonders bevorzugt ist ein erfindungsgemäßes Verfahren, wobei die genomische DNA aus einer DNA-Probe erhalten wurde, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Ein weitere Gegenstand der vorliegenden Erfindung ist eine Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Bevorzugt ist die Verwendung eines erfindungsgemäßen Verfahrens zur Unterscheidung von Zelltypen oder Geweben oder zur Untersuchung der Zelldifferenzierung.

Gegenstand der vorliegenden Erfindung ist ferner ein Kit, bestehend aus einem Bisulfit enthaltenen Reagenz, denaturierenden Reagenzien oder Lösungsmitteln, sowie Radikalfängern und optional Primern zur Herstellung der Amplifikate, sowie optional einer Anleitung zur Durchführung eines Assays nach einem der Ansprüche 1-28.

Beschrieben wird in der vorliegenden Erfindung ein Verfahren zur Herstellung doppelsträngiger DNA aus einer genomischen DNA-Probe, die nach Durchführung des Verfahrens die Eigenschaft hat, dass CG Basenpaare nur noch an den Positionen vorliegen, an denen sich zuvor in der genomischen DNA Methylcytosinbasen befanden. Nachfolgend können jegliche Verfahren verwendet werden, die chemische, biologische oder physikalische Eigenschaften der CG Basenpaare ausnutzen, um die Positionen, an denen sich in der genomischen DNA-Probe Methylcytosinbasen befanden, kenntlich zu machen. Es werden dazu auch im folgenden viele bevorzugte Ausführungsvarianten aufgeführt, jedoch wird es unterstellt, dass der Fachmann von diesen abgeleitet oder alternativ weitere naheliegenden Möglichkeiten finden mag, CG Basenpaarungen nachzuweisen und für den hier vorgegebenen Zweck zu nutzen.

Die in dem Verfahren eingesetzte genomische DNA wird bevorzugt aus einer DNA-Probe erhalten, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Das Verfahren zur Detektion von Cytosin-Methylierung in genomischer DNA wird durch folgende Schritte charakterisiert: Zuerst setzt man eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird. Anschließend wird im zweiten Schritt die vorbehandelte DNA unter Verwendung einer Polymerase und mindestens einem Primeroligonukleotid amplifiziert. Im dritten Schritt wird das Amplifikat erneut mit einem Reagenz chemisch umgesetzt, wobei 5-Methylcytosin unverändert bleibt und Cytosin in Uracil oder eine andere im Basenpaarungsverhalten dem Uracil ähnliche Base umgewandelt wird. Im letzten Schritt werden die in dem Amplifikat nach der erneuten chemischen Behandlung verbleibenden Cytosinbasen und/oder Guaninbasen nachgewiesen.

In einer besonders bevorzugten Variante wird das Verfahren durch die folgenden Schritte charakterisiert: Das Verfahren zur Detektion von Cytosin-Methylierung in genomischer DNA wird durch folgende Schritte charakterisiert: Zuerst setzt man eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird. Anschließend wird im zweiten Schritt die vorbehandelte DNA unter Verwendung einer Polymerase und mindestens einem Primeroligonukleotid amplifiziert. Im dritten Schritt wird das Amplifikat erneut mit einem Reagenz chemisch umgesetzt, wobei 5-Methylcytosin unverändert bleibt und Cytosin in Uracil oder eine andere im Basenpaarungsverhalten dem Uracil ähnliche Base umgewandelt wird. Im vierten Schritt werden die chemisch behandelten Amplifikate aus dem dritten Schritt erneut amplifiziert. Im letzten Schritt werden die in dem Amplifikat nach der erneuten chemischen Behandlung verbleibenden Cytosinbasen und/oder Guaninbasen nachgewiesen.

Die chemische Behandlung erfolgt besonders bevorzugt mit einer Lösung eines Hydrogensulfits (= Disulfit, Bisulfit). Diese Behandlung führt unter geeigneten Bedingungen, wie dem Fachmann an sich bekannt, zu einer Addition des Hydrogensulfits an nicht methylierte Cytosinbasen. Auf diese Reaktion folgt eine praktisch quantitative Umwandlung der Aminofunktion des Cytosins in eine Ketofunktion, so dass ein sulfoniertes Uracil entsteht, welches durch alkalische Hydrolyse in Uracil überführt wird. Es erfolgt also auf diesem Wege eine selektive Umwandlung der nicht methylierten Cytosinbasen in Uracil, während die 5-Methylcytosine unverändert bleiben. Diese Reaktion findet nur statt, wenn die DNA einzelsträngig vorliegt. In einer besonders bevorzugten Variante des Verfahrens erfolgt daher die chemische Behandlung der DNA derart, dass diese in Agarose eingebettet vorliegt. Dadurch wird die Ausbildung von Duplexes verhindert.

In einer weiteren besonders bevorzugten Variante des Verfahrens wird bei der chemischen Behandlung ein Lösungsmittel oder ein Reagenz zugesetzt, welches die DNA-Duplex denaturiert. Bei diesen Lösungsmitteln oder Reagenzien handelt es sich bevorzugt um Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF.

Bevorzugt ist es zudem, einen Radikalfänger zuzusetzen, welcher den Abbau der DNA unter den Reaktionsbedingungen verhindert. Bei diesen Radikalfängern handelt es sich bevorzugt um Di-, Trihydroxybenzole, green tea extract, pine bark extract (Pycnogenol), Ginkgo Biloba extract (EGb 761), a flavonoid blend of several fruit and vegetable extracts (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-carboxylic acid), 2,6-Di-tert-butylphenol, 4-Methyl-di-tert-butylphenol, 4-Methoxy-di-tert-butylphenol, 2, 6-Di-tert-butyl-p-cresol, 3, 4-Dihydroxybenzoesäure, Vitamin C, E, Q, Hydrochinon, Ubichinon, Lignane, Hydroxyterpene, Flavonoide, Curcumin, Tannine, Retinsäureverbindungen, Ge-132 Bisbetacarboxyethyl germanium sesquioxide, Superoxid dismutase (SOD), Superoxid katalase, Alpha-Naphthoflavone, Ginkgo biloba extract (EGb 761), Di(2-methyl-5-chlorophenyl)dithionate und Cu(II)-Derivate, Mebendazole, CS (Chloroform soluble) alkaloidal extract, 4-(3,5-Di-tert-butyl-4-hydroxyphenyl)-3-hydroxy-1,2-naphthochinon, 4-(3,5-Di-tert-butyl-4-hydroxyphenyl)-3-methoxy-1, 2-naphthochinon, 4-(3,5-Di-tert-butyl-4-hydroxyphenyl)-1,2-naphthochinon, 2-(3,5-Di-tert-butyl-4-hydroxyphenyl)-3-brom-1,4-naphthochinon, 2-(3, 5-Di-tert-butyl-4-hydroxyphenyl)-3-chlor-1,4-naphthochinon, 2-(3, 5-Di-tert-butyl-4-hydroxyphenyl)-3-methoxy-1,4-naphthochinon, 2- (3, 5-Di-tert-butyl-4-hydroxyphenyl) -3-hydroxy-1,4-naphthochinon, 2-(3,5-Di-tert-butyl-4-hydroxyphenyl)-1,4-naphthochinon, 4-(3,5-Di-tert-butyl-4-hydroxyphenyl)-3-hydroxy-5,5,8,8-tetramethyl-5,6,7,8-tetrahydro-1,2-anthrachinon, 4-(3,5-Di-tert-butyl-4-hydroxyphenyl)-3-methoxy-5,5,8,8-tetramethyl-5,6,7,8-tetrahydro-1,2-anthrachinon, 4-(3,5-Di-tert-butyl-4-hydroxyphenyl)-5,5,8,8-tetramethyl-5,6,7,8-tetrahydro-1,2-anthrachinon, 3-Brom-4-(3,5-di-tert-butyl-4-hydroxyphenyl)-5,5,8,8-tetramethyl-5,6,7,8-tetrahydro-1,2-anthrachinon, 2-(3,5-Di-tert-butyl-4-oxocyclohexa-2,5-dienyliden)-indan-1,3-dion, 2-(3,5-Di-tert-butyl-4-oxocyclohexa-2,5-dienyliden)-3,4-epoxy-3-hydroxy-4-methoxy-3,4-dihydro-2H-naphthalin-1-on, 2-(3,5-Di-tert-butyl-4-oxocyclohexa-2,5-dienyliden)-3,4-epoxy-3,4-dimethoxy-3,4-dihydro-2H-naphthalin-1-on, 2-(3,5-Di-tert-butyl-4-hydroxyphenyl)-indan-1-on, 3,3-Bi-[2-(3,5-di-tert-butyl-4-hydroxyphenyl)-inden-1-on]-3-yl, 2-(3,5-Di-tert-butyl-4-hydroxyphenyl)-3-brom-5,5,8,8-tetramethyl-5,6,7,8-tetrahydro-1,4-anthrachinon, 2-(3,5-Di-tert-butyl-4-hydroxyphenyl)-3-chlor-5,5,8,8-tetramethyl-5,6,7,8-tetrahydro-1,4-anthrachinon, 2-(3,5-Di-tert-butyl-4-hydroxyphenyl)-3-methoxy-5,5,8,8-tetramethyl-5,6,7,8-tetrahydro-1,4-anthrachinon, 2-(3, 5-Di-tert-butyl-4-hydroxyphenyl)-3-hydroxy-5,5,8,8-tetramethyl-5,6,7,8-tetrahydro-1,4-anthrachinon, 2-(3, 5-Di-tert-butyl-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-5,5,8,8-tetramethyl-5,6,7,8-tetrahydro-1,4-anthrachinon, 2-Brom-3-(3,5-dibrom-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-3-hydroxy-5,5,8,8-tetramethyl-5,6,7,8-tetrahydro-1,4-anthrachinon, 3-Brom-2-(3,5-di-tert-butyl-4-hydroxyphenyl)-1,4-anthrachinon, 2-(3, 5-Di-tert-butyl-4-hydroxyphenyl)-3-methoxy-1,4-anthrachinon, 2-(3,5-Di-tert-butyl-4-hydroxyphenyl)-3-hydroxy-1,4-anthrachinon, 5,5,8,8-Tetramethyl-5,6,7,8-tetrahydronaphthalin-1,3-diol, 3-Methoxy-5,5,8,8-tetramethyl-5,6,7,8-tetrahydronaphthalin-1-ol, 4-(3-Chlor-5,5,8,8-tetramethyl-1,4-dioxo-1,4,5,6,7,8-hexahydroanthracen-2-yl)-benzoesäure, Methyl-4-(3-chlor-5,5,8,8-tetramethyl-1,4-dioxo-1,4,5,6,7,8-hexahydroanthracen-2-yl)-benzoat, 4-(3-Hydroxy-1,4-dioxo-1,4-dihydronaphthalin-2-yl)-benzoesäure, Methyl-(3-methoxy-1,4-dioxo-1,4-dihydronaphthalin-2-yl)-benzoesäure, 4-(3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat, 4-(3-Hydroxy-5,5,8,8-tetramethyl-1,4-dioxo-1,4,5,6,7,8-hexahydroanthracen-2-yl-azo)-benzoesäure, 3-(3,5-Di-tert-butyl-4-oxocyclohexa-2,5-dienyliden)-5,5,8,8-tetramethyl-5,6,7,8-tetrahydrocyclopenta[b]naphthalin-1,2-dion, 3-(3, 5-Di-tert-butyl-4-oxocyclohexa-2,5-dienyliden)-5,5,8,8-tetramethyl-5,6,7,8-tetrahydroanthracen-3H-1,2,4-trion, 2-(3,5-Di-tert-butyl-4-hydroxyphenyl)-3-methoxy-5,8-dimethyl-1,4-naphthochinon, 2-(3,5-Di-tert-butyl-4-hydroxyphenyl)-3-methoxy-6,7-dimethyl-1,4-naphthochinon, 2-(3,5-Di-tert-butyl-4-hydroxyphenyl)-3-methoxy-5-methyl-1,4-naphthochinon, 2-(3,5-Di-tert-butyl-4-hydroxyphenyl)-2-methoxy-5-methyl-1,4-naphthochinon, 2-(3,5-Di-tert-butyl-4-hydroxyphenyl)-3-methoxy-6-methyl-1,4-naphthochinon, 3-(3,5-Di-tert-butyl-4-hydroxyphenyl)-2-methoxy-6-methyl-1,4-naphthochinon, 2-(3,5-Di-tert-butyl-4-hydroxyphenyl)-3-methoxy-5,6-dimethyl-1,4-naphthochinon, 3-(3,5-Di-tert-butyl-4-hydroxyphenyl)-2-methoxy-5,6-dimethyl-1,4-naphthochinon, 2-(3,5-Di-tert-butyl-4-hydroxyphenyl)-3-methoxy-5,7-dimethyl-1,4-naphthochinon, 3-(3,5-Di-tert-butyl-4-hydroxyphenyl)-2-methoxy-5,7-dimethyl-1,4-naphthochinon, 2-(3,5-Di-tert-butyl-4-hydroxyphenyl)-3-ethylthio-5-methyl-1,4-naphthochinon, 2-(3,5-Di-tert-butyl-4-hydroxyphenyl)-3-ethylthio-6-methyl-1,4-naphthochinon, 2-(3,5-Di-tert-butyl-4-hydroxyphenyl)-3-hydroxy-5,8-dimethyl-1,4-naphthochinon, 2-(3,5-Di-tert-butyl-4-hydroxyphenyl)-3-hydroxy-6,7-dimethyl-1,4-naphthochinon,2-(3,5-Di-tert-butyl-4-hydroxyphenyl) -3-hydroxy-5-methyl-1, 4-naphthochinon, 3-(3,5-Di-tert-butyl-4-hydroxyphenyl)-2-hydroxy-5-methyl-1,4-naphthochinon, 2-(3,5-Di-tert-butyl-4-hydroxyphenyl)-3-hydroxy-6-methyl-1,4-naphthochinon, 3-(3,5-Di-tert-butyl-4-hydroxyphenyl)-2-hydroxy-6-methyl-1,4-naphthochinon, 2-(3,5-Di-tert-butyl-4-hydroxyphenyl)-3-hydroxy-5,6-dimethyl-1,4-naphthochinon, 2-(3-Brom-5-tert-butyl-4-hydroxyphenyl)-3-hydroxy-5,6-dimethyl-1,4-naphthochinon, 3-(3,5-Di-tert-butyl-4-hydroxyphenyl)-2-hydroxy-5,6-dimethyl-1,4-naphthochinon, 2-(3,5-Di-tert-butyl-4-hydroxyphenyl)-3-hydroxy-5,7-dimethyl-1,4-naphthochinon, 3-(3,5-Di-tert-butyl-4-hydroxyphenyl)-2-hydroxy-5,7-dimethyl-1,4-naphthochinon.

Bevorzugt ist zudem eine Variante des Verfahrens, in welcher die Reagenzien, die für die chemische Behandlung der DNA eingesetzt werden, vor der nachfolgenden Amplifikation ganz oder teilweise abgetrennt werden. Dies kann beispielsweise durch Bindung der DNA an eine Festphase und Entfernen der Reagenzien durch Waschschritte durchgeführt werden. In einer besonders bevorzugten Variante des Verfahrens wird die Probe nach der chemischen Behandlung jedoch lediglich verdünnt, so dass die Reagenzien in einer derart niedrigen Konzentration vorliegen, dass sie die unmittelbar nachfolgende Amplifikation nicht stören.
In einer weiteren besonders bevorzugten Variante des Verfahrens wird die Amplifikation von mehreren DNA-Abschnitten gleichzeitig in einem Reaktionsgefäß durchführt. Besonders bevorzugt wird zudem eine hitzebeständige DNA-Polymerase verwendet und die PCR für die Amplifikation eingesetzt, wie es dem Fachmann im Prinzip bekannt ist.

Bevorzugt ist es zudem, jeweils vor den Amplifikationen eine Desulfonierung der DNA durchzuführen, da selbige aufgrund der chemischen Vorbehandlung noch über Sulfonsäuregruppen verfügen kann. Besonders bevorzugt erfolgt diese Desulfonierung in einem basischen Puffer, und idealerweise handelt es sich bei diesem basischen Puffer um den Puffer, der auch für die Amplifikationsreaktion eingesetzt wird.

Im folgenden werden nun mehrere, dem Fachmann überwiegend im Prinzip geläufige Verfahren beschrieben, wie die nach der Durchführung des Verfahrens verbleibenden Cytosin bzw. Guaninbasen, die nunmehr als Indikatoren für das Vorliegen von Cytosin-Methylierung in der ursprünglichen genomischen DNA-Probe dienen, nachgewiesen werden können.

Besonders bevorzugt werden die verbleibenden Cytosin- und/oder Guaninbasen bzw. Cytosin-Guanin Basenpaare durch Hybridisierungsreaktionen nachgewiesen. Bevorzugt kann man diesen Nachweis derart ausführen, dass die Amplifikationsprodukte an einen Oligonukleotid Array hybridisiert werden und man anschliessend die folgenden Teilschritte ausführt: Im ersten Schritt wird die amplifizierte DNA an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind. Das Oligonukleotid oder die Oligonukleotide mit jeweils bekannter Sequenz von n Nukleotiden werden nun im nächsten Schritt mittels einer Polymerase mindestens um ein Nukleotid verlängert, wobei das Nukleotid eine nachweisbare Markierung trägt und die Verlängerung vom Methylierungsstatus des jeweiligen Cytosins in der ursprünglichen genomischen DNA-Probe abhängt. So ist es beispielsweise möglich, dass nur dann eine Verlängerungsreaktion stattfindet, wenn an das 3'-Ende des Oligonukleotids auf der Template-DNA ein Guanin angrenzt, das den Einbau eines markierten Cytosins erlaubt. Die Anknüpfung des markierten Cytosins an das Oligonukleotid dient dann letztendlich als Nachweisreaktion für das Vorliegen eines methylierten Cytosins in der ursprünglichen genomischen Probe.

Weiterhin ist es möglich, dass die nach der zweiten chemischen Behandlung (und optionalen zweiten Amplifikation) verbleibenden Cytosin- und/oder Guaninbasen durch Sequenzierungsreaktionen nachgewiesen werden. Dem Fachmann sind vor allem die Sequenzierung nach Sanger und die Sequenzierung nach Maxam-Gilbert geläufig. Vor allem letztere bietet sich hier an, da in der Duplex nur noch dort CG Basenpaare vorliegen, wo sich zuvor ein Methylcytosin in der ursprünglichen genomischen DNA-Probe befand. Es ist also möglich, durch die C und/oder die G spezifischen Spaltungsreaktionen Fragmente zu erzeugen, deren Grösse unmittelbar von dem Methylierungszustand der Probe anhängen. Diese Fragmente können dann beispielsweise auf Sequenziergelen oder durch Kapillarelektrophorese hinsichtlich ihrer Grösse bestimmt werden. Gegenüber einer nur einfachen Bisulfitbehandlung, wie sie im Stand der Technik beschrieben wird, spielt das hier beschriebene Verfahren in dieser Variante auch den besonderen Vorteil aus, dass die Spaltung des jeweiligen Gegenstranges nicht erfolgen kann, da dieser entweder kein C oder kein G enthalten kann. Dadurch ist die Zahl der entstehenden Fragmente deutlich reduziert und auch, falls mehrere Amplifikate gleichzeitig untersucht werden, eine viel übersichtlichere Auswertung möglich.

Bevorzugt ist zudem eine Variante des Verfahrens, in der für die Detektion der vorbehandelten DNA die Amplifikationsprodukte an einen Oligonukleotid Array hybridisiert werden und anschliessend die folgenden Teilschritte ausgeführt werden: Im ersten Schritt hybridisiert man einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist. Im zweiten Schritt wird das Oligonukleotid der ersten Spezies mit bekannter Sequenz von n Nukleotiden mittels einer Polymerase um höchstens die Anzahl von Nukleotiden verlängert, die zwischen dem 3' -Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt. Beispielsweise ist es möglich, dass eine Verlängerung des 3'-Endes nur dann erfolgt, wenn in der chemisch vorbehandelten DNA ein Thymin anstelle eines Cytosins vorliegt, da beispielsweise in dem verwendeten Nukleotidmix Guanin nur als Terminator vorhanden ist und damit die Verlängerung, falls ein Cytosin vorliegt, unterbrochen wird. Ein Cytosin wiederum ist nach der chemischen Behandlung nur dann zugegen, wenn an der betreffenden Position in der ursprünglichen DNA-Probe ein 5-Methylcytosin vorlag.

Im letzten Schritt inkubiert man die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

Bevorzugt ist zudem ein Verfahren, wobei für die Detektion der vorbehandelten DNA die PCR-Produkte auf einen Oligonukleotid Array hybridisiert werden und anschliessend die folgenden Teilschritte ausgeführt werden: Im ersten Schritt hybridisiert man die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind. Im zweiten Schritt wird das Oligonukleotid, sofern es mit seinem 3'Terminus zuvor ohne Basenfehlpaarungen an die zu untersuchenden Position hybridisierte, mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt. So kann beispielsweise eine Verlängerung nur dann stattfinden, wenn die genomische DNA an der Position, an die das oben genannte Oligonukleotid mit seinem 3'-Ende bindet, ursprünglich unmethyliert vorlag, weil sonst nach der chemischen Vorbehandlung an dieser Position ein Cytosin verbleibt, welches nach der Bindung des Oligonukleotids einen Mismatch am 3'-Ende verursacht welche die Verlängerung unterbindet. Liegt keine Methylierung vor, so kann der Primer dagegen ohne Mismatch mit dem 3'-Ende an die Template-DNA binden.

Die Analyse der entstandenen DNA-Oligomere, die zum Nachweis der Cytosin-Methylierung dienen, kann beispielsweise über Fluoreszenzmarkierungen erfolgen, welche über markierte Nucleotide in die DNA eingebaut werden.

Dem Fachmann ist zudem geläufig, dass man die meisten der beschriebenen Nachweisreaktionen auch derart ausführen kann, dass beteiligte Oligonukleotide an eine Festphase gebunden sind.

So ist es beispielsweise besonders bevorzugt möglich, die PCR nach der zweiten chemischen Behandlung derart auszuführen, dass radioaktiv oder fluoreszent markierte C und/oder G Nukleotide eingebaut werden und zudem einer der PCR-Primer an eine Festphase gebunden ist. Nach der Entfernung aller nicht an die Festphase gebundenen Edukte und Produkte verbleibt ein an die Festphase gebundenes PCR-Produkt, in welchem die Anzahl der eingebauten Markierungen proportional ist zur Anzahl der der nach der zweiten chemischen Behandlung verbliebenen Guanin- und/oder Cytosinbasen und damit unmittelbar von der Anzahl der Cytosin-Methylierungen in der genomischen Probe abhängt. Damit ist eine direkte Quantifizierung der methylierten Cytosine in diesem PCR-Produkt möglich.

Besonders bevorzugt ist ein Verfahren, wobei die PCR-Produkte und/oder Verlängerungsprodukte und/oder Ligationsprodukte für die Detektion mit einer nachweisbaren Markierung versehen sind.

Besonders bevorzugt sind diese Markierungen Fluoreszenzmarkierungen, Radionuklide oder ablösbare Massenmarkierungen, die in einem Massenspektrometer nachgewiesen werden, oder Kombinationen davon.

In einer weiteren bevorzugten Variante des Verfahrens werden die PCR-Produkte und/oder Verlängerungsprodukte und/oder Ligationsprodukte insgesamt im Massenspektrometer nachgewiesen und sind somit durch ihre Masse eindeutig charakterisiert. In einer weiteren besonders bevorzugten Variante des Verfahrens wird jeweils ein Fragment der PCR-Produkte und/oder Verlängerungsprodukte und/oder Ligationsprodukte im Massenspektrometer nachgewiesen. Bevorzugt werden diese Fragmente der PCR-Produkte und/oder Verlängerungsprodukts und/oder Ligationsprodukts durch Verdau mit einer oder mehrerer Exo- oder Endonukleasen erzeugt. In einer wiederum besonders bevorzugten Ausführung des Verfahrens weisen die erzeugten Fragmente zur besseren Detektierbarkeit im Massenspektrometer eine einzelne positive oder negative Nettoladung auf.

Besonders bevorzugt werden die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

Gegenstand der vorliegenden Erfindung ist zudem die Verwendung des beschriebenen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Weiterhin besonders bevorzugt ist die Verwendung des Verfahrens zur Unterscheidung von Zelltypen oder Geweben oder zur Untersuchung der Zelldifferenzierung.

Gegenstand der vorliegenden Erfindung ist zudem ein Kit, bestehend aus einem Bisulfit enthaltenen Reagenz, denaturierenden Reagenzien oder Lösungsmitteln, sowie Radikalfängern und/oder Primern zur Herstellung der Amplifikate, sowie optional einer Anleitung zur Durchführung des erfindungsgemässen Verfahrens.

Die folgenden Beispiele erläutern die Erfindung ohne diese einzuschränken.

### Beispiel 1:

Veränderung einer genomischen Sequenz durch zweifache Behandlung mit Bisulfit

Als Beispiel zur Erläuterung des Verfahrens soll hier die folgende doppelsträngige DNA-Sequenz dienen, die ein potentiell methyliertes CG Dinukleotid enthält:
A 3'-TGATCTAGCATGACTAC-5*'*
B 5'-ACTAGATCGTACTGATG-3'

Die doppelsträngige Sequenz kann verwendet werden, um nachteilige Ereignisse einer bestimmten Kategorie für Patienten oder Individuen zu untersuchen. Man setzt eine genomische, unbekannt methylierte DNA-Probe (20 ng), die mit einem Restriktionsenzym, hier Mss1, verdaut wurde, ein. Die verdaute DNA wird mit Hydrogensulfit (Bisulfit, Disulfit) und einem Radikalfänger bei erhöhter Temperatur chemisch umgewandelt. Ein Reagenz oder Lösungsmittel, welches die Denaturierung unterstützt, wird zugesetzt. Die DNA wird zuerst thermisch denaturiert und anschließend mit Bisulfit, dem Radikalfänger und dem denaturierenden Reagenz versetzt und längere Zeit bei erhöhter Temperatur inkubiert. Die Bisulfitreaktion führt zur Umwandlung aller nicht methylierten Cytosinbasen in Uracil. Zur Reinigung der Bisulfit behandelten DNA wird diese auf eine Reversed Phase C18 Festphase gebunden und durch Waschen mit einer geeigneten Pufferlösung von Chemikalien befreit. Anschließend wird die DNA mit einem polaren Lösungsmittel wie z. B. Acetonitril oder Wasser eluiert und auf ein kleineres Volumen konzentriert. Die alkalische Hydrolyse der mit Bisulfit behandelten Fragmente erfolgt unmittelbar vor der spezifischen Amplifikation unter basischen Bedingungen. Danach wird die Probe unter Verwendung von je 25 pmol an spezifischen Primeroligonukleotiden (enthalten keine CG Dinukleotide) vervielfältigt. Nach der ersten Behandlung mit einer Lösung eines Hydrogensulfits, wobei eine Umwandlung der nicht methylierten Cytosinbasen in Uracil erfolgt, und nachfolgender PCR, die die nach der chemischen Behandlung mit Hydrogensulfit nicht mehr komplementären Stränge amplifiziert und zugleich Uracil im wesentlichen durch Thymin ersetzt, ergeben sich die folgenden doppelsträngigen DNA-Sequenzen:
A) für den Fall, dass CG (+) methyliert vorlag:

| aus dem unteren Strang B: | | |
|---|---|---|
| C | (+) 3'-TAATCTAGCATAACTAC-5' | |
| B' | (+) 5'-ATTAGATCGTATTGATG-3' | Sequenz L (up) |
| | | |

| aus dem oberen Strang A: | | |
|---|---|---|
| A' | (+) 3'-TGATTTAGCATGATTAT-5' | |
| D | (+) 5'-ACTAAATCGTACTAATA-3' | Sequenz U(up) |

B) für den Fall, dass CG (-) unmethyliert vorlag:

| aus dem unteren Strang B: | | |
|---|---|---|
| C | (-) 3'-TAATCTAACATAACTAC-5' | |
| B' | (-) 5'-ATTAGATTGTATTGATG-3' | Sequenz L(down) |
| | | |

| aus dem oberen Strang A: | | |
|---|---|---|
| A' | (-) 3'-TGATTTAGTATGATTAT-5' | |
| D | (-) 5'-ACTAAATCATACTAATA-3' | Sequenz U(down) |

Die zweite Behandlung Hydrogensulfit wird analog der ersten durchgeführt. Die Amplifikate werden zuerst thermisch denaturiert und dabei durch Zugabe eines Reagenzes oder Lösungsmittels unterstützt, dann chemisch umgesetzt und anschließend amplifiziert, wobei die alkalische Hydrolyse wiederum kurz vor der Amplifikation durchgeführt wird. Die zweite spezifische Amplifikation kann mit einem Teil der Bisulfit behandelten Probe durchgeführt werden. Die Reaktionslösung kann so lange verdünnt werden, dass die in der Hydrogensulfitreaktion benötigten Chemikalien die PCR nicht mehr stören. Andererseits kann die Probe wieder mittels einer Reversed Phase C18 Festphase gereinigt werden. Die doppelt hydrogensulfitbehandelte DNA wird unter Verwendung von je 25 pmol an spezifischen Primeroligonukleotiden (enhalten keine CG Dinukleotide) vervielfältigt. Dabei müssen die Sequenzen der Primeroligonukleotide der ersten und zweiten Amplifikation nicht übereinstimmen.

Nach der zweiten Behandlung mit einer Lösung eines Hydrogensulfits, wobei wiederum eine Umwandlung der nicht methylierten Cytosinbasen in Uracil erfolgt, und nachfolgender PCR, die die nach der chemischen Behandlung mit Hydrogensulfit nicht mehr komplementären Stränge amplifiziert und zugleich Uracil im wesentlichen durch Thymin ersetzt, ergeben sich nunmehr die folgenden doppelsträngigen DNA-Sequenzen:
A) für den Fall, dass CG (+) methyliert vorlag:

| aus dem unteren Strang B' und C: | | |
|---|---|---|
| | | |
| E | (+) 3*'*-TAATCTAACATAACTAC-5*'* | Sequenz LL(up) |
| B" | (+) 5'-ATTAGATTGTATTGATG-3' | |
| | | |
| C' | (+) 3'-TAATTTAGTATAATTAT-5' | |
| F | (+) 5'-ATTAAATCATATTAATA-3' | Sequenz LU(up) |

aus dem oberen Strang D und A':

| | | |
|---|---|---|
| G | (+) 3'-TAATTTAACATAATTAT-5' | |
| D' | (+) 5'-ATTAAATTGTATTAATA-3' | Sequenz UZ(up) |
| | | |
| H | (+) 3'-TGATTTAGTATGATTAT-5' | |
| A '' | (+) 5'-ACTAAATCATACTAATA-3' | Sequenz UU(up) |

B) für den Fall, dass CG (-) unmethyliert vorlag:

| aus dem unteren Strang C und B': | | |
|---|---|---|
| | | |
| E | (-) 3'-TAATCTAACATAACTAC-5 | |
| B' ' | (-) 5'-ATTAGATTGTATTGATG-3' | Sequenz LL(down) |
| | | |
| C' | (-) 3*'*-TAATTTAATATAATTAT-5*'* | |
| F | (-) 5*'*-ATTAAATTATATTAATA-3' | Sequenz LU(down) |
| | | |

| aus dem oberen Strang D und A': | | |
|---|---|---|
| G | (-) 3'-TAATTTAATATAATTAT-5' | |
| D' | (-) 5'-ATTAAATTATATTAATA-3' | Sequenz UL(down) |
| | | |
| H | (-) 3'-TGATTTAGTATGATTAT-5' | |
| A'' | (-) 5'-ACTAAATCATACTAATA-3' | Sequenz UU(down) |

Die Sequenzen UU sind unabhängig vom Methylierungsstatus der Probe identisch; das gleiche gilt für die Sequenzen LL. Somit sind beide Sequenzen für die Bestimmung des Methylierungsstatus nicht verwendbar. LU und UL jedoch unterscheiden sich an den ehemaligen CG Positionen in Abhängigkeit vom Methylierungsstatus der DNA-Probe (in den Sequenzen hervorgehoben). Bemerkenswert und der eigentlich Vorteil der Methode ist insbesondere, dass in den jeweiligen Strängen Cytosin bzw. Guanin nur dann auftauchen, wenn zuvor eine Methylierung vorlag. Damit liegt praktisch eine Übersetzung der Methylcytosinbasen in der genomischen Probe in eine auch für den Doppelstrang charakteristische Base vor, während bei der einfachen Bisulfit-Behandlung die Base jeweils nur in einem der beiden Stränge für Methylcytosin charakteristisch ist. Dies erlaubt nun die Anwendung praktisch aller, dem Fachmann bekannten Methoden zur Sequenzierung (wie zum Beispiel Sequenzierung nach Maxam-Gilbert) oder zur Genotypisierung, ohne dass auf den Gegenstrang Rücksicht genommen werden muss. Wird beispielsweise eine sequenzspezifische Spaltung der nach der zweiten Bisulfit-Behandlung amplifizierten DNA an Cytosinbasen durchgeführt, so erfolgt nur eine Spaltung des einen Stranges, der andere bleibt unverändert.

### Beispiel 2: Veränderung einer genomischen Sequenz durch zweifache Behandlung mit Bisulfit

Als Beispiel wurde das Gen HSMDR1 des humanen Genoms gewählt. Im Experiment wurden nach einer ersten Bisulfitbehandlung DNA-Fragmente der Länge 2010 bp dieses Gens erzeugt und gleichzeitig gc-reiche Domänen am 5'- und 3'-Ende generiert. Da diese "künstlichen" Enden am 5'-und 3'-Ende der PCR-Produkte nach der ersten Bisulfitbehandlung erzeugt wurden, unterliegen sie in der zweiten Bisulfitbehandlung einer einfachen Umwandlung. Dies führt zu einer höheren Spezifität dieser Domänen, da sie (im Gegensatz zur übrigen Sequenz) noch C's und G's enthalten. Damit eignen sie sich besonders für das Design von Primern für eine Amplifikation nach der zweiten Bisulfitbehandlung. Die Amplifikation eines kurzen Stückes der Ziel-DNA erfolgt mit Primern die ausschließlich aus A's und T's bestehen. Die Sequenzen der genomischen, einfach bisulfitbehandelten und doppelt bisulfitbehandelten DNA sind am Ende dargestellt.

### Beschreibung der Figuren:

### Figur 1:

Schema zur Herstellung von PCR-Produkt mit gc-Domaine aus einfach bisulfitbehandelter DNA:
a) 5'gtgatcccgggcgagctcccTAAGTATGTTGAAGAAAGATTATTG;
b) 3'gcttgggctgcaggtcgaccTTTTAACCTTCTATCTCATCAAC;
c) bisulfitbehandelte DNA, Einzelstrang

### Figur 2:

Genomische Sequenz der Umgebung des HSMDR1-Gens: LOCUS HSMDR1A2932 bp DNA humanes MDR1 (multidrug resistance) Gen für das P-Glycoprotein

### Figur 3:

Sequenz des oberen Strangs nach der ersten Bisulfitbehandlung für den Fall vollständiger Aufmethylierung der CG's (PCR-Produkt mit Primer 1 und 2)

### Figur 4:

Sequenzen des unteren Strangs nach der zweiten Bisulfitbehandlung für den Fall vollständiger Aufmethylierung aller CG's
a) PCR-Produkt mit Primer 3 und 4 (154 bp)
b) PCR-Produkt mit Primer 5 und 6 (2010 bp)

### Vorgehen:

a)Restriktion von humaner DNA unbekannten Methylierungsstatus mit Mss1
b)Bisulfitbehandlung mit der Agarosemethode
c)Bisulfit-spezifische PCR zur Herstellung von MDR1-Fragmenten einer Länge 1936bp. Dabei werden gleichzeitig mittels Domaine-Primern "künstliche" gc-reiche Enden an die PCR-Produkte generiert
d)Zweite Bisulfitbehandlung ohne Agarose mit anschließender ZipTip-Aufreinigung
e)Amplifikation der doppelt bisulfitbehandelten DNA mittels Primern für die gc-Domaine

### Verwendete Primer:

Bisulfit Primer, Produktgröße:2010 bp

1 MDR1-B-U-gc gtgatcccgggcgagctcccTAAGTATGTTGAAGAAAGATTATTG

2 MDR1-B-L-gc gcttgggctgcaggtcgacCTTTTAACCTTCTATCTCATCAAC

<--gc-domaine-------><-genspezifische Sequenz-->

| *Primer für doppelt bisulfitierte DNA ohne gc-Domaine, Produkt-größe: 154 bp* | | |
|---|---|---|
| 3 | MDR1-2B-U3 | tttttttttatttttttattat |
| 4 | MDR1-2B-L3 | atttttttttattattttttaat |
| | | |

| *Primer für doppelt bisulfitierte DNA mit gc-Domaine, Produkt-größe: 2010 bp* | | |
|---|---|---|
| 5 6 | MDR1-2B-L-gc MDR1-2B-U-gc | GTTTGGGTTGTAGGTTGAT ataatcccaaacaaactccc |

### Versuchsbedingungen:

Die Restriktion von humaner genomische DNA (Fa. Promega) erfolgte mit Mss1 (Fa. Fermentas) nach Herstellerangaben.

Die erste Bisulfitbehandlung der verdauten DNA erfolgte mit der Agarosemethode, wie sie im Stand der Technik beschrieben ist.

Die Amplifikation von DNA erfolgte unter folgenden PCR-Bedingungen (FA. Qiagen):
- 1 µl DNA (10 ng bisulfitbehandelte DNA)
- 0.2 µl Taq (1 Unit)
- 0,2 µl dNTP (25 mM each) 0,25 mM final
- 1 µl primer1 (12,5 pmol/µl) 0,5 pmol/µl final
- 1 µl Primer2 (12,5 pmol/µl) 0,5 pmol/µl final
- 2,5 µl 10fach PCR-Puffer
- 19,1 H₂O (molecular grade)

Folgende Cycler-Programme wurden verwendet:
- PCR mit Domäneprimern (Primer1 und 2) nach erster Bisulfitbehandlung:
   95°C/20:00; 95°C/1:00; 56 °C/0:45; 72°C/2:00; cycles:40; 72°C/10:00; 4°C/end
- PCR nach zweiter Bisulfitbehandlung(Primer 3 und 4):
   95°C/20:00; 95°C/1:00; 40 °C/0:45; 72°C/1:00; cycles:40; 72°C/10:00; 4°C/end
- PCR nach zweiter Bisulfitbehandlung(Primer 5 und 5):
   95°C/20:00; 95°C/1:00; 56 °C/0:45; 72°C/2:00; cycles:40; 72°C/10:00; 4°C/end

Die zweite Bisulfitbehandlung des hergestellten PCR-Productes erfolgte ohne Agarose in Lösung . Zur Entfernung der Salze und Chemikalien wurde die doppelt bisulfitierte DNA mit Hilfe der ZipTip®-Methode (FA: Millipore) aufgereinigt. Dabei wurde folgendermaßen vorgegangen:
- neue Spitze 3x mit 10 µl 2M TEAA Puffer gespühlt
- 35 µl PCR-Produkt aufgezogen
- 1x Spülen mit 10 µl 2M TEAA
- 3x Spülen mit 10 µl 0.1M TEAA
- 3x Spülen mit 10 µl Wasser (bidest.)
- Eluieren des Produktes mit 100 µl MeCN (10x10 µl frisches MeCN aufgezogen und in ein Tube eluiert
- 100 µl Eluat wurden unter Vacuum eingetrocknet, in 30 µl Wasser (bidest) resuspendiert und sofort in die PCR eingesetzt

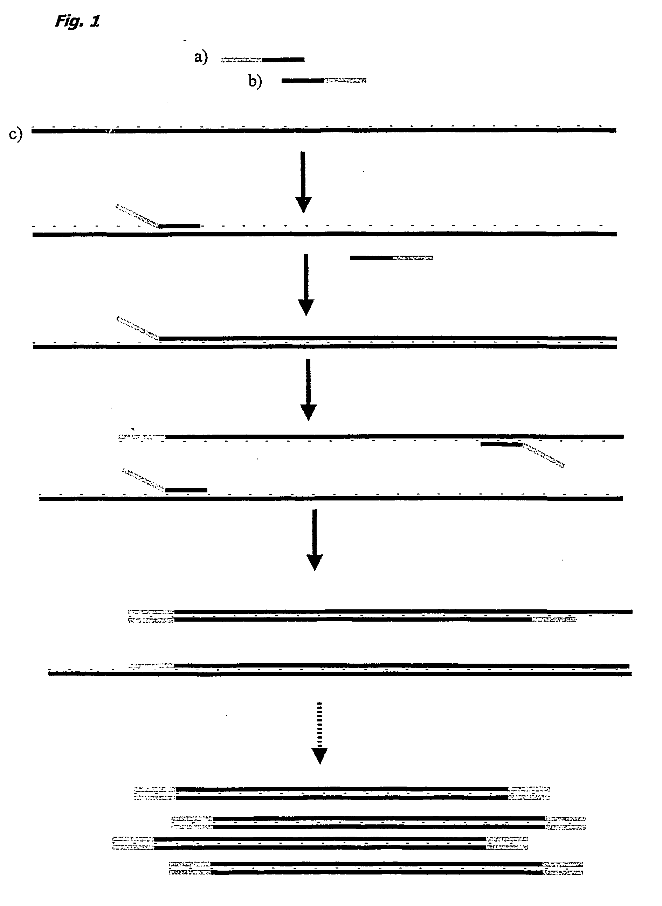

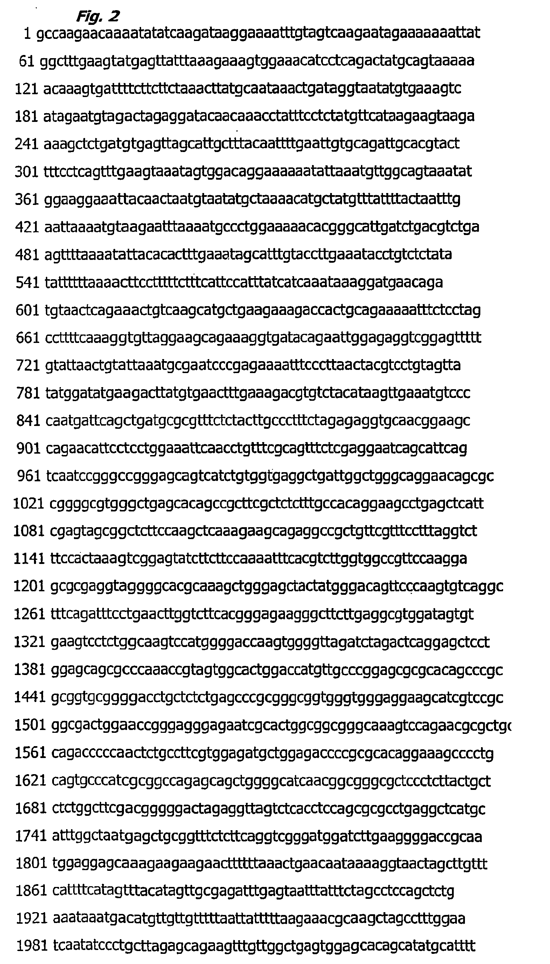

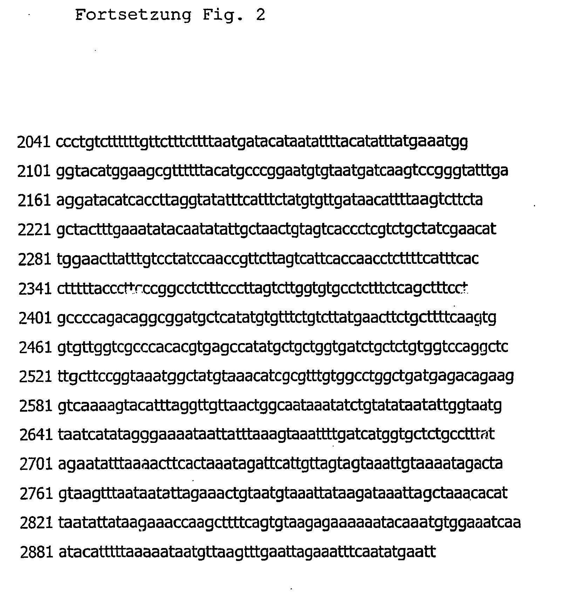

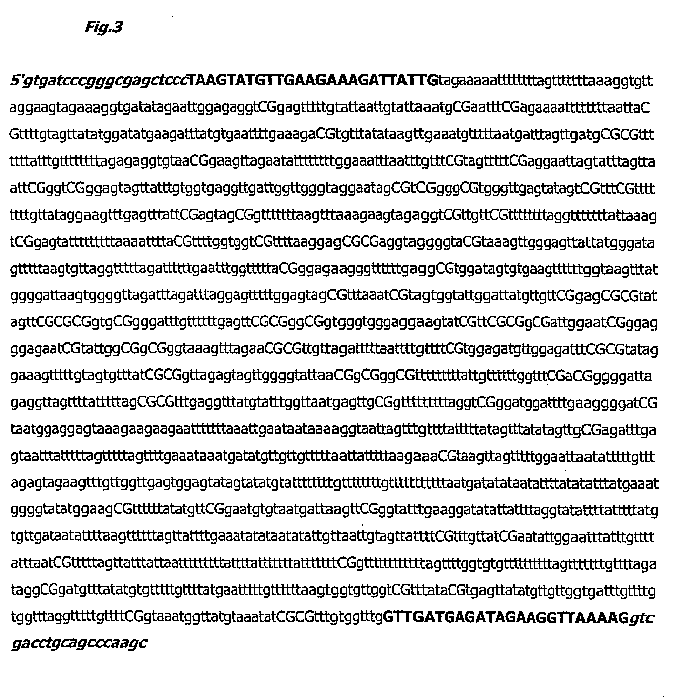

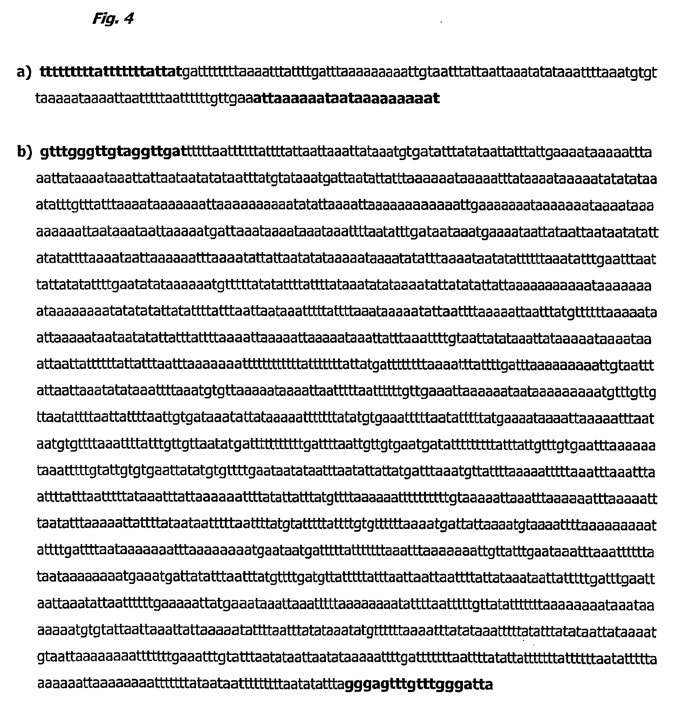

## Patentansprüche

1. Verfahren zur Detektion von Cytosin-Methylierung in genomischer DNA, **dadurch gekennzeichnet, dass** man die folgenden Verfahrensschritte ausführt:
a) man setzt eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird;
b) man amplifiziert die vorbehandelte DNA unter Verwendung einer Polymerase und mindestens eines Primeroligonukleotids;
c) man setzt das Amplifikat erneut mit einem Reagenz chemisch um, wobei 5-Methylcytosin unverändert bleibt und Cytosin in Uracil oder eine andere im Basenpaarungsverhalten dem Uracil ähnliche Base umgewandelt wird;
d) man weist die in dem Amplifikat nach der erneuten chemischen Behandlung verbleibenden Cytosinbasen und/oder Guaninbasen nach.

2. Verfahren zur Detektion von Cytosin-Methylierung in genomischer DNA nach Anspruch 1, **dadurch gekennzeichnet, dass** man die folgenden Verfahrensschritte ausführt:
a) man setzt eine genomische DNA-Probe mit einem Reagenz chemisch um, wobei 5-Methylcytosin unverändert bleibt und Cytosin in Uracil oder eine andere im Basenpaarungsverhalten dem Uracil ähnliche Base umgewandelt wird;
b) man amplifiziert die vorbehandelte DNA unter Verwendung einer Polymerase und mindestens eines Primeroligonukleotids;
c) man setzt das Amplifikat erneut mit einem Reagenz chemisch um, wobei 5-Methylcytosin unverändert bleibt und Cytosin in Uracil oder eine andere im Basenpaarungsverhalten dem Uracil ähnliche Base umgewandelt wird;
d) man amplifiziert die chemisch behandelten Amplifikate aus Schritt c) erneut;
e) man weist die in dem Amplifikat verbleibenden Cytosinbasen und/oder Guaninbasen nach.

3. Verfahren nach Anspruch 1 oder 2, **dadurch gekennzeichnet, dass** die chemische Behandlung mit Natriumbisulfit (=Hydrogensulfit, Disulfit) erfolgt.

4. Verfahren nach Anspruch 3, **dadurch gekennzeichnet, dass** die chemische Behandlung nach Einbetten der DNA in Agarose erfolgt.

5. Verfahren nach einem der voranstehenden Ansprüche, **dadurch gekennzeichnet, dass** bei der chemischen Behandlung ein die DNA-Duplex denaturierendes Reagenz und/oder ein Radikalfänger zugegen ist.

6. Verfahren nach Anspruch 5, **dadurch gekennzeichnet, dass** das denaturierende Reagenz aus einer der folgenden Substanzen ausgewählt ist:
Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylenglykol-dialkylether, Pentaethylenglykoldiakylether, Hexaethylenglykoldialkylether, DMSO oder THF.

7. Verfahren nach Anspruch 5 oder 6, **dadurch gekennzeichnet, dass** der Radikalfänger aus einer der folgenden Substanzen ausgewählt ist:
Di-, Trihydroxybenzole, green tea extract, pine bark extract (Pycnogenol), Ginkgo Biloba extract (EGb 761), a flavonoid blend of several fruit and vegetable extracts (GNLD), Bio-Normalizer (Sun-O Corp), DPPH (1,1-Diphenyl-2-picrylhydrazyl), NDGA (Nordihydroguajaret-säure), Trolox (6-Hydroxy-2,5,7,8-tetramethylchroman-2-carboxylic acid), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, E, Q, Hydrochinon, Ubichinon, Lignane, Hydroxyterpene, Flavonoide, Curcumin, Tannine, Retinsäureverbindungen, Ge-132 Bisbetacarboxyethyl germanium sesquioxide, Superoxid dismutase (SOD), Superoxid katalase, Alpha-Naphthoflavone, Ginkgo biloba extract (EGb 761), Di(2-methyl-5-chlorophenyl)dithionate und Cu(II)-Derivate, Mebendazole, CS (Chloroform soluble) alkaloidal extract, 4-(3,5-Di-tert-butyl-4-hydroxyphenyl)-3-hydroxy-1,2-naphthochinon, 4-(3,5-Di-tert-butyl-4-hydroxyphenyl)-3-methoxy-1,2-naphthochinon, 4-(3,5-Di-tert-butyl-4-hydroxyphenyl)-1,2-naphthochinon, 2-(3,5-Di-tert-butyl-4-hydroxyphenyl)-3-brom-1,4-naphthochinon, 2-(3,5-Di-tert-butyl-4-hydroxyphenyl)-3-chlor-1,4-naphthochinon, 2-(3,5-Di-tert-butyl-4-hydroxyphenyl)-3-methoxy-1,4-naphthochinon, 2-(3,5-Di-tert-butyl-4-hydroxyphenyl) -3-hydroxy-1,4-naphthochinon, 2-(3,5-Di-tert-butyl-4-hydroxyphenyl) -1,4-naphthochinon, 4-(3,5-Di-tert-butyl-4-hydroxyphenyl)-3-hydroxy-5,5,8,8-tetramethyl-5,6,7,8-tetrahydro-1,2-anthrachinon, 4-(3,5-Di-tert-butyl-4-hydroxyphenyl)-3-methoxy-5,5,8,8-tetramethyl-5,6,7,8-tetrahydro-1,2-anthrachinon, 4-(3,5-Di-tert-butyl-4-hydroxyphenyl)-5,5,8,8-tetramethyl-5,6,7,8-tetrahydro-1,2-anthrachinon, 3-Brom-4- (3,5-di-tert-butyl-4-hydroxyphenyl)-5,5,8,8-tetramethyl-5,6,7,8-tetrahydro-1,2-anthrachinon, 2-(3,5-Di-tert-butyl-4-oxocyclohexa-2,5-dienyliden)-indan-1,3-dion, 2-(3,5-Di-tert-butyl-4-oxocyclohexa-2,5-dienyliden)-3,4-epoxy-3-hydroxy-4-methoxy-3,4-dihydro-2H-naphthalin-1-on, 2-(3,5-Di-tert-butyl-4-oxocyclohexa-2,5-dienyliden)-3,4-epoxy-3,4-dimethoxy-3,4-dihydro-2H-naphthalin-1-on, 2-(3,5-Di-tert-butyl-4-hydroxyphenyl)-indan-1-on, 3,3-Bi-[2-(3,5-di-tert-butyl-4-hydroxyphenyl)-inden-1-on]-3-yl, 2-(3,5-Di-tert-butyl-4-hydroxyphenyl)-3-brom-5,5,8,8-tetramethyl-5,6,7,8-tetrahydro-1,4-anthrachinon, 2-(3,5-Di-tert-butyl-4-hydroxyphenyl)-3-chlor-5,5,8,8-tetramethyl-5,6,7,8-tetrahydro-1,4-anthrachinon, 2-(3,5-Di-tert-butyl-4-hydroxyphenyl)-3-methoxy-5,5,8,8-tetramethyl-5,6,7,8-tetrahydro-1,4-anthrachinon, 2-(3,5-Di-tert-butyl-4-hydroxyphenyl)-3-hydroxy-5,5,8,8-tetramethyl-5,6,7,8-tetrahydro-1,4-anthrachinon, 2-(3,5-Di-tert-butyl-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-5,5,8,8-tetramethyl-5,6,7,8-tetrahydro-1,4-anthrachinon, 2-Brom-3-(3,5-dibrom-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-3-hydroxy-5,5,8,8-tetramethyl-5,6,7,8-tetrahydro-1,4-anthrachinon, 3-Brom-2-(3,5-di-tert-butyl-4-hydroxyphenyl)-1,4-anthrachinon, 2-(3,5-Di-tert-butyl-4-hydroxyphenyl)-3-methoxy-1,4-anthrachinon, 2-(3,5-Di-tert-butyl-4-hydroxyphenyl)-3-hydroxy-1,4-anthrachinon, 5,5,8,8-Tetramethyl-5,6,7,8-tetrahydronaphthalin-1,3-diol, 3-Methoxy-5,5,8,8-tetramethyl-5,6,7,8-tetrahydronaphthalin-1-ol, 4-(3-Chlor-5,5,8,8-tetramethyl-1,4-dioxo-1,4,5,6,7,8-hexahydroanthracen-2-yl)-benzoesäure, Methyl-4-(3-chlor-5,5,8,8-tetramethyl-1,4-dioxo-1,4,5,6,7,8-hexahydroanthracen-2-yl)-benzoat, 4-(3-Hydroxy-1,4-dioxo-1,4-dihydronaphthalin-2-yl)-benzoesäure, Methyl-(3-methoxy-1,4-dioxo-1,4-dihydronaphthalin-2-yl)-benzoesäure, 4-(3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat, 4-(3-Hydroxy-5,5,8,8-tetramethyl-1,4-dioxo-1,4,5,6,7,8-hexahydroanthracen-2-yl-azo)-benzoesäure, 3-(3,5-Di-tert-butyl-4-oxocyclohexa-2,5-dienyliden)-5,5,8,8-tetramethyl-5,6,7,8-tetrahydrocyclopenta[b]naphthalin-1,2-dion, 3-(3, 5-Di-tert-butyl-4-oxocyclohexa-2,5-dienyliden)-5,5,8,8-tetramethyl-5,6,7,8-tetrahydroanthracen-3H-1,2,4-trion, 2-(3, 5-Di-tert-butyl-4-hydroxyphenyl)-3-methoxy-5,8-dimethyl-1,4-naphthochinon, 2-(3, 5-Di-tert-butyl-4-hydroxyphenyl)-3-methoxy-6,7-dimethyl-1,4-naphthochinon, 2-(3,5-Di-tert-butyl-4-hydroxyphenyl)-3-methoxy-5-methyl-1,4-naphthochinon, 2-(3, 5-Di-tert-butyl-4-hydroxyphenyl)-2-methoxy-5-methyl-1,4-naphthochinon, 2-(3,5-Di-tert-butyl-4-hydroxyphenyl)-3-methoxy-6-methyl-1,4-naphthochinon, 3-(3,5-Di-tert-butyl-4-hydroxyphenyl)-2-methoxy-6-methyl-1,4-naphthochinon, 2-(3,5-Di-tert-butyl-4-hydroxyphenyl)-3-methoxy-5,6-dimethyl-1,4-naphthochinon, 3-(3,5-Di-tert-butyl-4-hydroxyphenyl)-2-methoxy-5,6-dimethyl-1,4-naphthochinon, 2- (3,5-Di-tert-butyl-4-hydroxyphenyl)-3-methoxy-5,7-dimethyl-1,4-naphthochinon, 3-(3,5-Di-tert-butyl-4-hydroxyphenyl)-2-methoxy-5,7-dimethyl-1,4-naphthochinon, 2-(3,5-Di-tert-butyl-4-hydroxyphenyl)-3-ethylthio-5-methyl-1,4-naphthochinon, 2-(3,5-Di-tert-butyl-4-hydroxyphenyl)-3-ethylthio-6-methyl-1,4-naphthochinon, 2-(3,5-Di-tert-butyl-4-hydroxyphenyl)-3-hydroxy-5,8-dimethyl-1,4-naphthochinon, 2-(3,5-Di-tert-butyl-4-hydroxyphenyl)-3-hydroxy-6,7-dimethyl-1,4-naphthochinon, 2-(3,5-Di-tert-butyl-4-hydroxyphenyl)-3-hydroxy-5-methyl-1,4-naphthochinon, 3-(3,5-Di-tert-butyl-4-hydroxyphenyl)-2-hydroxy-5-methyl-1,4-naphthochinon, 2-(3,5-Di-tert-butyl-4-hydroxyphenyl)-3-hydroxy-6-methyl-1,4-naphthochinon, 3-(3,5-Di-tert-butyl-4-hydroxyphenyl)-2-hydroxy-6-methyl-1,4-naphthochinon, 2-(3,5-Di-tert-butyl-4-hydroxyphenyl)-3-hydroxy-5,6-dimethyl-1,4-naphthochinon, 2-(3-Brom-5-tert-butyl-4-hydroxyphenyl)-3-hydroxy-5,6-dimethyl-1,4-naphthochinon, 3-(3,5-Di-tert-butyl-4-hydroxyphenyl)-2-hydroxy-5,6-dimethyl-1,4-naphthochinon, 2-(3, 5-Di-tert-butyl-4-hydroxyphenyl)-3-hydroxy-5,7-dimethyl-1,4-naphthochinon, 3-(3,5-Di-tert-butyl-4-hydroxyphenyl)-2-hydroxy-5,7-dimethyl-1,4-naphthochinon.

8. Verfahren nach einem der voranstehenden Ansprüche, **dadurch gekennzeichnet, dass** man Reagenzien, die für die chemische Behandlung der DNA eingesetzt werden, vor der jeweils nachfolgenden Amplifikation ganz oder teilweise abtrennt.

9. Verfahren nach einem der voranstehenden Ansprüche, **dadurch gekennzeichnet, dass** man die Probe nach der chemischen Behandlung vor der Amplifikation verdünnt.

10. Verfahren nach einem der voranstehenden Ansprüche, **dadurch gekennzeichnet, dass** man die Amplifikation von mehreren DNA-Abschnitten gleichzeitig in einem Reaktionsgefäß durchführt.

11. Verfahren nach einem der voranstehenden Ansprüche, **dadurch gekennzeichnet, dass** man für die Amplifikation eine hitzebeständige DNA-Polymerase verwendet.

12. Verfahren nach einem der voranstehenden Ansprüche, **dadurch gekennzeichnet, dass** man vor den Amplifikationen in den Ansprüchen 1 oder 2 eine Desulfonierung der DNA durchführt.

13. Verfahren nach einem der Ansprüche 1 bis 12, **dadurch gekennzeichnet, dass** man die nach der zweiten chemischen Behandlung verbleibenden Cytosin- und/oder Guaninbasen durch Hybridisierungsreaktionen nachweist.

14. Verfahren nach einem der Ansprüche 1 bis 12, **dadurch gekennzeichnet, dass** man die nach der zweiten chemischen Behandlung verbleibenden Cytosin- und/oder Guaninbasen durch spezifischen Einbau von nachweisbaren Markierungen an den Cytosin- und/oder Guaninbasen nachweist und/oder quantifiziert.

15. Verfahren nach einem der Ansprüche 1 bis 12, **dadurch gekennzeichnet, dass** man in Schritt d) gemäß Anspruch 1 oder Schritt e) gemäß Anspruch 2 für die Detektion der vorbehandelten DNA die Amplifikationsprodukte an einen Oligonukleotid Array hybridisiert und man anschliessend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) man verlängert das Oligonukleotid mit bekannter Sequenz von n Nukleotiden mittels einer Polymerase mindestens um ein Nukleotid, wobei das Nukleotid eine nachweisbare Markierung trägt und die Verlängerung vom Methylierungsstatus des jeweiligen Cytosins in der genomischen DNA-Probe abhängt.

16. Verfahren nach einem der Ansprüche 1 bis 12, **dadurch gekennzeichnet, dass** man die nach der zweiten chemischen Behandlung verbleibenden Cytosin- und/oder Guaninbasen durch Sequenzierungsreaktionen nachweist.

17. Verfahren nach einem der Ansprüche 1 bis 12, **dadurch gekennzeichnet, dass** man in Schritt d) gemäß Anspruch 1 oder Schritt e) gemäß Anspruch 2 für die Detektion der vorbehandelten DNA die Amplifikationsprodukte an einen Oligonukleotid Array hybridisiert und man anschliessend die folgenden Teilschritte ausführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen, die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) man verlängert das Oligonukleotid der ersten Spezies mit bekannter Sequenz von n Nukleotiden mittels einer Polymerase um höchstens die Anzahl von Nukleotiden, die zwischen dem 3' -Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man **dadurch** ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

18. Verfahren nach einem der Ansprüche 1-12, **dadurch gekennzeichnet, dass** man für die Detektion der vorbehandelten DNA gemäß Schritt d) in Anspruch 1 die PCR-Produkte auf einen Oligonukleotid Array hybridisiert und man anschliessend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) man verlängert das Oligonukleotid, sofern es mit seinem 3' Terminus zuvor ohne Basenfehlpaarungen an die zu untersuchenden Position hybridisierte, mittels einer Polymerase mindestens um ein Nukleotid, wobei mindestens ein Nukleotid eine nachweisbare Markierung trägt und die Verlängerung vom Methylierungsstatus des jeweiligen Cytosins in der genomischen DNA-Probe abhängt.

19. Verfahren nach einem der voranstehenden Ansprüche, **dadurch gekennzeichnet, dass** man die PCR-Produkte und/oder Verlängerungsprodukte und/oder Ligationsprodukte für die Detektion mit einer nachweisbaren Markierung versieht.

20. Verfahren nach einem der voranstehenden Ansprüche, **dadurch gekennzeichnet, dass** die Markierungen Fluoreszenzmarkierungen sind.

21. Verfahren nach einem der voranstehenden Ansprüche, **dadurch gekennzeichnet, dass** die Markierungen Radionuklide sind.

22. Verfahren nach einem der Ansprüche 1 bis 19, **dadurch gekennzeichnet, dass** die Markierungen ablösbare Massenmarkierungen sind, die in einem Massenspektrometer nachgewiesen werden.

23. Verfahren nach einem der voranstehenden Ansprüche, **dadurch gekennzeichnet, dass** bei einer der Amplifikationen einer der Primer an eine Festphase gebunden ist.

24. Verfahren nach einem der Ansprüche 1 bis 19, **dadurch gekennzeichnet, dass** man die PCR-Produkte und/oder Verlängerungsprodukte und/oder Ligationsprodukte insgesamt im Massenspektrometer nachweist und somit durch ihre Masse eindeutig charakterisiert sind.

25. Verfahren nach einem der Ansprüche 1 bis 19, **dadurch gekennzeichnet, dass** man jeweils ein Fragment der PCR-Produkte und/oder Verlängerungsprodukte und/oder Ligationsprodukte im Massenspektrometer nachweist.

26. Verfahren nach Anspruch 25, **dadurch gekennzeichnet, dass** man das Fragment des PCR-Produkts und/oder Verlängerungsprodukts und/oder Ligationsprodukts durch Verdau mit einer oder mehrerer Exo- oder Endonukleasen erzeugt.

27. Verfahren nach Anspruch 25 oder 26, **dadurch gekennzeichnet, dass** zur besseren Detektierbarkeit im Massenspektrometer die erzeugten Fragmente eine einzelne positive oder negative Nettoladung aufweisen.

28. Verfahren nach einem der Ansprüche 1 bis 19, **dadurch gekennzeichnet, dass** man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

29. Verfahren nach einem der voranstehenden Ansprüche, wobei die genomische DNA aus einer DNA-Probe erhalten wurde, wobei Quellen für DNA z. B. Zelllinien, Blut, Sputum, Stuhl, Urin, Gehirn-Rückenmarks-Flüssigkeit, in Paraffin einbettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

30. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

31. Verwendung eines Verfahrens nach einem der Ansprüche 1 bis 29 zur Unterscheidung von Zelltypen oder Geweben oder zur Untersuchung der Zelldifferenzierung.

## Claims

1. A method for the detection of cytosine methylation in genomic DNA, hereby **characterized in that** the following method steps are conducted:
a) a genomic DNA sample is chemically converted with a reagent, whereby 5-methylcytosine remains unchanged and cytosine is converted to uracil or another base similar to uracil in its base pairing behavior;
b) the pretreated DNA is amplified with the use of a polymerase and at least one primer oligonucleotide;
c) the amplified product is again chemically converted with a reagent, whereby 5-methylcytosine remains unchanged and cytosine is converted to uracil or another base similar to uracil in its base pairing behavior;
d) the cytosine bases and/or guanine bases remaining in the amplified product after the repeated chemical treatment are detected.

2. The method for the detection of cytosine methylation in genomic DNA according to claim 1, further **characterized in that** the following method steps are conducted:
a) a genomic DNA sample is chemically converted with a reagent, whereby 5-methylcytosine remains unchanged and cytosine is converted to uracil or another base similar to uracil in its base pairing behavior;
b) the pretreated DNA is amplified with the use of a polymerase and at least one primer oligonucleotide;
c) the amplified product is again chemically converted with a reagent, whereby 5-methylcytosine remains unchanged and cytosine is converted to uracil or another base similar to uracil in its base pairing behavior;
d) the chemically treated amplified product from step c) is again amplified;
e) the cytosine bases and/or guanine bases remaining in the amplified product are detected.

3. The method according to claim 1 or 2, further **characterized in that** the chemical treatment is conducted with sodium bisulfite (= hydrogen sulfite, disulfite).

4. The method according to claim 3, further **characterized in that** the chemical treatment is conducted after embedding the DNA in agarose.

5. The method according to one of the preceding claims, further **characterized in that** a reagent that denatures the DNA duplex and/or a radical trap is (are) present in the chemical treatment.

6. The method according to claim 5, further **characterized in that** the denaturing reagent is selected from one of the following substances: polyethylene glycol dialkyl ether, dioxane and substituted derivatives, urea or derivatives, acetonitrile, primary alcohols, secondary alcohols, tertiary alcohols, diethylene glycol dialkyl ether, triethylene glycol dialkyl ether, tetraethylene glycol dialkyl ether, pentaethylene glycol dialkyl ether, hexaethylene glycol dialkyl ether, DMSO or THF.

7. The method according to claim 5 or 6, further **characterized in that** the radical trap is selected from one of the following substances:
di- and trihdroxybenzenes, green tea extract, pine bark extract (Pycnogenol), Ginkgo Biloba extract (EGb 761), a flavonoid blend of several fruit and vegetable extracts (GNLD), Bio-Normalizer (Sun-O Corp), DPPH (1,1-diphenyl-2-picrylhydrazyl), NDGA (nordihydroguaiaretic acid), Trolox (6-hydroxy-2,5,7,8-tetramethylchromane 2-carboxylic acid), 2,6-di-tert-butylphenol, 4-methyl-di-tertbutylphenol, 4-methoxy-di-tert-butylphenol, 2,6-di-tert-butyl-p-cresol, 3,4-dihydroxybenzoic acid, vitamins C, E, Q, hydroquinone, ubiquinone, lignans, hydroxyterpenes, flavonoids, curcumin, tannins, retinoic acid compounds, Ge-132 bisbetacarboxyethyl germanium sesquioxides, superoxide dismutase (SOD), superoxide catalase, alpha-naphthoflavone, Ginkgo biloba extract (EGb 761), di-(2-methyl-5-chlorophenyl)dithionate and Cu(II) derivatives, mebendazole, CS (chloroform-soluble) alkaloidal extract, 4-(3,5-di-tert-butyl-4-hydroxyphenyl) -3-hydroxy-1,2-naphthoquinone, 4-(3,5-di-tert-butyl-4-hydroxyphenyl)-3-methoxy-1,2-naphthoquinone, 4-(3,5-di-tert-butyl-4-hydroxyphenyl)-1,2-naphthoquinone, 2-(3,5-di-tert-butyl-4-hydroxyphenyl)-3-bromo-1,4-naphthoquinone, 2-(3,5-di-tert-butyl-4-hydroxyphenyl)-3-chloro-1,4-naphthoquinone, 2-(3,5-di-tert-butyl-4-hydroxyphenyl)-3-methoxy-1,4-naphthoquinone, 2-(3,5-di-tert-butyl-4-hydroxyphenyl)-3-hydroxy-1,4-naphthoquinone, 2-(3,5-di-tert-butyl-4-hydroxyphenyl)-1,4-naphthoquinone, 4-(3,5-di-tert-butyl-4-hydroxyphenyl)-3-hydroxy-5,5,8,8-tetramethyl-5,6,7,8-tetrahydro-1,2-anthraquinone, 4-(3,5-di-tert-butyl-4-hydroxyphenyl)-3-methoxy-5,5,8,8-tetramethyl-5,6,7,8-tetrahydro-1,2-anthraquinone, 4-(3,5-di-tert-butyl-4-hydroxyphenyl)-5,5,8,8-tetramethyl-5,6,7,8-tetrahydro-1,2-anthraquinone, 3-bromo-4-(3,5-di-tert-butyl-4-hydroxyphenyl)-5,5,8,8-tetramethyl-5,6,7,8-tetrahydro-1,2-anthraquinone, 2-(3,5-di-tert-butyl-4-oxocyclohexa-2,5-dienylidene)-indane-1,3-dione, 2-(3,5-di-tert-butyl-4-oxocyclohexa-2,5-dienylidene)-3,4-epoxy-3-hydroxy-4-methoxy-3,4-dihydro-2H-naphthalene-1-one, 2-(3,5-di-tert-butyl-4-oxocyclohexa-2,5-dienylidene)-3,4-epoxy-3,4-dimethoxy-3,4-dihydro-2H-naphthalene-1-one, 2-(3,5-di-tert-butyl-4-hydroxyphenyl)-indane-1-one, 3,3-bi-[2-(3,5-di-tert-butyl-4-hydroxyphenyl)-indene-1-on]-3-yl, 2-(3,5-di-tert-butyl-4-hydroxyphenyl)-3-bromo-5,5,8,8-tetramethyl-5,6,7,8-tetrahydro-1,4-anthraquinone, 2-(3,5-di-tert-butyl-4-hydroxyphenyl)-3-chloro-5,5,8,8-tetramethyl-5,6,7,8-tetrahydro-1,4-anthraquinone, 2-(3,5-di-tert-butyl-4-hydroxyphenyl)-3-methoxy-5,5,8,8-tetramethyl-5,6,7,8-tetrahydro-1,4-anthraquinone, 2-(3,5-di-tert-butyl-4-hydroxyphenyl)-3-hydroxy-5,5,8,8-tetramethyl-5, 6, 7, 8-tetrahydro-1,4-anthraquinone, 2-(3,5-di-tert-butyl-4-hydroxyphenyl)-5,5,8,8-tetramethyl-5,6,7,8-tetrahydro-1,4-anthraquinone, 2-bromo-3-(3-bromo-5-tert-butyl-4-hydroxyphenyl)-5,5,8,8-tetramethyl-5,6,7,8-tetrahydro-1,4-anthraquinone, 2-bromo-3-(3,5-dibromo-4-hydroxyphenyl)-5,5,8,8-tetramethyl-5,6,7,8-tetrahydro-1,4-anthraquinone, 2-bromo-3-(3-bromo-5-tert-butyl-4-hydroxyphenyl)-3-hydroxy-5,5,8,8-tetramethyl-5,6,7,8-tetrahydro-1,4-anthraquinone, 3-bromo-2-(3,5-di-tert-butyl-4-hydroxyphenyl)-1,4-anthraquinone, 2-(3,5-di-tert-butyl-4-hydroxyphenyl)-3-methoxy-1,4-anthraquinone, 2-(3,5-di-tert-butyl-4-hydroxyphenyl)-3-hydroxy-1,4-anthraquinone, 5 , 5 , 8 , 8-tetramethyl-5 , 6 , 7 , 8-tetrahydronaphthalene-1,3-diol, 3-methoxy-5 , 5 , 8 , 8-tetramethyl-5 , 6 , 7 , 8-tetrahydronaphthalene-1-ol, 4-(3-chloro-5,5,8,8-tetramethyl-1,4-dioxo-1,4,5,6,7,8-hexahydroanthracen-2-yl) benzoic acid, methyl-4-(3-chloro-5,5,8,8-tetramethyl-1,4-dioxo-1,4,5,6,7,8-hexahydroanthracen-2-yl) benzoate, 4-(3-hydroxy-1,4-dioxo-1,4-dihydronaphthalen-2-yl) benzoic acid, methyl-(3-methoxy-1,4-dioxo-1,4-dihydronaphthalen-2-yl) benzoic acid, 4-(3-hydroxy-5,5,8,8-tetramethyl-1,4-dioxo-1,4,5,6,7,8-hexahydroanthracen-2-yl) benzoic acid, methyl-4-(3-hydroxy-1,4-dioxo-1,4-dihydronaphthalen-2-yl-azo) benzoate, 4-(3-hydroxy-5,5,8,8-tetramethyl-1,4-dioxo-1,4,5,6,7,8-hexahydroanthracen-2-yl-azo) benzoic acid, 3-(3,5-di-tert-butyl-4-oxocyclohexa-2,5-dienylidene)-5,5,8,8-tetramethyl-5,6,7,8-tetrahydrocyclopenta[b]naphthalene-1,2-dione, 3-(3,5-di-tert-butyl-4-oxocyclohexa-2,5-dienylidene)-5, 5, 8, 8-tetramethyl-5, 6, 7, 8-tetrahydroanthracene-3H-1,2,4-trione, 2-(3, 5-di-tert-butyl-4-hydroxyphenyl)-3-methoxy-5,8-dimethyl-1,4-naphthoquinone, 2-(3, 5-di-tert-butyl-4-hydroxyphenyl)-3-methoxy-6,7-dimethyl-1,4-naphthoquinone, 2-(3,5-di-tert-butyl-4-hydroxyphenyl)-3-methoxy-5-methyl-1,4-naphthoquinone, 2-(3,5-di-tert-butyl-4-hydroxyphenyl)-2-methoxy-5-methyl-1,4-naphthoquinone, 2-(3,5-di-tert-butyl-4-hydroxyphenyl)-3-methoxy-6-methyl-1,4-naphthoquinone, 3-(3,5-di-tert-butyl-4-hydroxyphenyl)-2-methoxy-6-methyl-1,4-naphthoquinone, 2-(3,5-di-tert-butyl-4-hydroxyphenyl)-3-methoxy-5,6-dimethyl-1,4-naphthoquinone, 3-(3, 5-di-tert-butyl-4-hydroxyphenyl)-2-methoxy-5,6-dimethyl-1,4-naphthoquinone, 2-(3,5-di-tert-butyl-4-hydroxyphenyl)-3-methoxy-5,7-dimethyl-1,4-naphthoquinone, 3-(3, 5-di-tert-butyl-4-hydroxyphenyl)-2-methoxy-5,7-dimethyl-1,4-naphthoquinone, 2-(3,5-di-tert-butyl-4-hydroxyphenyl)-3-ethylthio-5-methyl-1,4-naphthoquinone, 2-(3,5-di-tert-butyl-4-hydroxyphenyl)-3-ethylthio-6-methyl-1,4-naphthoquinone, 2-(3,5-di-tert-butyl-4-hydroxyphenyl)-3-hydroxy-5,8-dimethyl-1,4-naphthoquinone, 2-(3,5-di-tert-butyl-4-hydroxyphenyl)-3-hydroxy-6,7-dimethyl-1,4-naphthoquinone, 2-(3,5-di-tert-butyl-4-hydroxyphenyl)-3-hydroxy-5-methyl-1,4-naphthoquinone, 3-(3,5-di-tert-butyl-4-hydroxyphenyl)-2-hydroxy-5-methyl-1,4-naphthoquinone, 2-(3,5-di-tert-butyl-4-hydroxyphenyl)-3-hydroxy-6-methyl-1,4-naphthoquinone, 3-(3,5-di-tert-butyl-4-hydroxyphenyl)-2-hydroxy-6-methyl-1,4-naphthoquinone, 2-(3,5-di-tert-butyl-4-hydroxyphenyl)-3-hydroxy-5,6-dimethyl-1,4-naphthoquinone, 2-(3-bromo-5-tert-butyl-4-hydroxyphenyl)-3-hydroxy-5,6-dimethyl-1,4-naphthoquinone, 3-(3,5-di-tert-butyl-4-hydroxyphenyl)-2-hydroxy-5,6-dimethyl-1,4-naphthoquinone, 2-(3,5-di-tert-butyl-4-hydroxyphenyl)-3-hydroxy-5,7-dimethyl-1,4-naphthoquinone, 3-(3,5-di-tert-butyl-4-hydroxyphenyl)-2-hydroxy-5,7-dimethyl-1,4-naphthoquinone.

8. The method according to one of the preceding claims, further **characterized in that** the reagents which are used for the chemical treatment of DNA are completely or partially eliminated prior to the subsequent amplification.

9. The method according to one of the preceding claims, further **characterized in that** the samples are diluted after the chemical treatment, prior to the amplification.

10. The method according to one of the preceding claims, further **characterized in that** the amplification of several DNA segments is conducted simultaneously in one reaction vessel.

11. The method according to one of the preceding claims, further **characterized in that** a heat-stable DNA polymerase is used for the amplification.

12. The method according to one of the preceding claims, further **characterized in that** a desulfonation of the DNA is conducted prior to the amplifications in claims 1 or 2.

13. The method according to one of claims 1 to 12, further **characterized in that** the cytosine and/or guanine bases that remain after the second chemical treatment are detected by hybridization reactions.

14. The method according to one of claims 1 to 12, further **characterized in that** the cytosine and/or guanine bases that remain after the second chemical treatment are detected and/or quantified by specific incorporation of detectable labels in the cytosine and/or guanine bases.

15. The method according to one of claims 1 to 12, further **characterized in that** in step d) according to claim 1 or step e) according to claim 2 for the detection of the pretreated DNA, the amplification products are hybridized to an oligonucleotide array and then the following substeps are conducted: a) the amplified genomic DNA is hybridized to at least one oligonucleotide with the formation of a duplex, whereby said hybridized oligonucleotide lies directly adjacent at its 3'-end, or at a distance of up to 10 bases, to the positions which are to be investigated relative to their methylation in the genomic DNA sample;
b) the oligonucleotide with known sequence of n nucleotides is extended by means of a polymerase by at least one nucleotide, whereby the nucleotide bears a detectable label and the extension of the respective cytosine in the genomic DNA sample depends on the methylation state.

16. The method according to one of claims 1 to 12, further **characterized in that** the cytosine and/or guanine bases that remain after the second chemical treatment are detected by sequencing reactions.

17. The method according to one of claims 1 to 12, further **characterized in that** in step d) according to claim 1 or step e) according to claim 2 for the detection of the pretreated DNA, the amplification products are hybridized to an oligonucleotide array and then the following substeps are conducted:
(a) a set of oligonucleotides is hybridized to the amplified genomic DNA with the formation of a duplex, wherein this set of oligonucleotides is comprised of two different species and wherein the hybridized oligonucleotides of the first species lie directly adjacent at their 3'-end, or at a distance of up to 10 bases, to the positions which are to be investigated relative to their methylation in the genomic DNA sample and whereby the second oligonucleotide of the second species hybridizes to a second region of the target molecule, so that the 5'-end of the oligonucleotide of the second species is separated by a gap of the magnitude of a single nucleotide or up to 10 nucleotides from the 3'-end of the hybridized oligonucleotide of the first species at the site of said selected position;
(b) the oligonucleotide of the first species with known sequence of n nucleotides is extended by means of a polymerase by at most the number of nucleotides which lie between the 3'-end of the oligonucleotide of the first species and the 5'-end of the oligonucleotide of the second species, wherein the extension depends on the methylation state of the respective cytosine in the genomic DNA sample;
(c) the oligonucleotide is incubated in the presence of a ligase, whereby the oligonucleotide of the first species and the oligonucleotide of the second species that lie next to one another and that have been extended by the polymerase reaction are joined and a ligation product is obtained in this way, as long as an extension of the oligonucleotide of the first species has been produced in the preceding step, so that now the 3'-end with the present 3'-hydroxy function of the extended oligonucleotide is directly adjacent to the 5'-end of the oligonucleotide of the second species.

18. The method according to one of claims 1 to 12, further **characterized in that** according to step d) in claim 1, for the detection of the pretreated DNA, the PCR products are hybridized to an oligonucleotide array and then the following substeps are conducted:
(a) the amplified genomic DNA is hybridized to at least one oligonucleotide with known sequence of n nucleotides with the formation of a duplex, whereby said hybridized oligonucleotides with their 3'-end hybridize partially or completely to the positions which are to be investigated with respect to their methylation in the genomic DNA sample;
b) the oligonucleotide, insofar as it has previously hybridized by its 3'-terminus without erroneous base pairing to the position to be investigated, is extended by means of a polymerase by at least one nucleotide, whereby at least one nucleotide bears a detectable label and the extension of the respective cytosine in the genomic DNA sample depends on the methylation state.

19. The method according to one of the preceding claims, further **characterized in that** the PCR products and/or extension products and/or ligation products are provided with a detectable label for the detection.

20. The method according to one of the preceding claims, further **characterized in that** the labels are fluorescent labels.

21. The method according to one of the preceding claims, further **characterized in that** the labels are radionuclides.

22. The method according to one of claims 1 to 19, further **characterized in that** the labels are removable mass labels which are detected in a mass spectrometer.

23. The method according to one of the preceding claims, further **characterized in that** in one of the amplifications, one of the primers is bound to a solid phase.

24. The method according to one of claims 1 to 19, further **characterized in that** the PCR products and/or extension products and/or ligation products overall are detected in the mass spectrometer and thus are clearly **characterized by** their mass.

25. The method according to one of claims 1 to 19, further **characterized in that** a fragment of the PCR products and/or extension products and/or ligation products is detected each time in the mass spectrometer.

26. The method according to claim 25, further **characterized in that** the fragment of the PCR product and/or extension product and/or ligation product is produced by digestion with one or more exo- or endonucleases.

27. The method according to claim 25 or 26, further **characterized in that** the generated fragments have a single positive or negative net charge for better detectability in the mass spectrometer.

28. The method according to one of claims 1 to 19, further **characterized in that** the PCR products and/or extension products and/or ligation products are detected and visualized by means of matrix-assisted laser desorption/ionization mass spectrometry (MALDI-TOF) or by means of electrospray mass spectrometry (ESI).

29. The method according to one of the preceding claims, wherein the genomic DNA has been obtained from a DNA sample, whereby sources for DNA include, e.g., cell lines, blood, sputum, stool, urine, cerebrospinal fluid, tissue embedded in paraffin, for example tissue from eyes, intestine, kidney, brain, heart, prostate, lungs, breast or liver, histological slides and all possible combinations thereof.

30. Use of a method according to one of the preceding claims for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical, psychological and social consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as a consequence of an abnormality in the development process; malfunction, damage or disorder of the skin, the muscles, the connective tissue or the bones; endocrine and metabolic malfunction; headaches or sexual malfunctions.

31. Use of the method according to one of claims 1 to 29 for distinguishing cell types or tissues or for investigating cell differentiation.

## Revendications

1. Procédé pour la détection d'une méthylation de la cytosine dans un ADN génomique, **caractérisé en ce que** l'on met en oeuvre les étapes suivantes :
a) on fait chimiquement réagir un échantillon d'ADN génomique avec un réactif, la 5-méthylcytosine restant non modifiée et la cytosine étant convertie en uracile ou en une autre base analogue à l'uracile pour ce qui est de son comportement en appariement de bases ;
b) on amplifie l'ADN prétraité en utilisant une polymérase et au moins un oligonucléotide amorce ;
c) on fait chimiquement réagir de nouveau l'amplifiat avec un réactif, la 5-méthylcytosine restant non modifiée et la cytosine étant convertie en uracile ou en une autre base analogue à l'uracile pour ce qui est de son comportement en appariement de bases ;
d) on détecte les bases cytosines et/ou les bases guanines restant dans l'amplifiat après le nouveau traitement chimique.

2. Procédé pour la détection d'une méthylation de la cytosine dans un ADN génomique selon la revendication 1, **caractérisé en ce que** l*'*on met en oeuvre les étapes suivantes :
a) on fait chimiquement réagir un échantillon d'ADN génomique avec un réactif, la 5-méthylcytosine restant non modifiée et la cytosine étant convertie en uracile ou en une autre base analogue à l'uracile pour ce qui est de son comportement en appariement de bases ;
b) on amplifie l'ADN prétraité en utilisant une polymérase et au moins un oligonucléotide amorce ;
c) on fait chimiquement réagir de nouveau l'amplifiat avec un réactif, la 5-méthylcytosine restant non modifiée et la cytosine étant convertie en uracile ou en une autre base analogue à l'uracile pour ce qui est de son comportement en appariement de bases ;
d) on amplifie de nouveau l'amplifiat chimiquement traité de l'étape c) ;
e) on détecte les bases cytosines et/ou les bases guanines restant dans l'amplifiat.

3. Procédé selon la revendication 1 ou 2, **caractérisé en ce que** le traitement chimique est effectué avec du bisulfite de sodium (= hydrogénosulfite, disulfite).

4. Procédé selon la revendication 3, **caractérisé en ce que** le traitement chimique est effectué après enrobage de l'ADN dans de l'agarose.

5. Procédé selon l'une des revendications précédentes, **caractérisé en ce qu'**un réactif dénaturant le duplex d'ADN et/ou un fixateur de radicaux sont présents lors du traitement chimique.

6. Procédé selon la revendication 5, **caractérisé en ce que** le réactif dénaturant est choisi parmi les substances suivantes :
les éthers dialkyliques du polyéthylèneglycol, le dioxanne et ses dérivés substitués, l'urée ou ses dérivés, l'acétonitrile, les alcools primaires, les alcools secondaires, les alcools tertiaires, les éthers dialkyliques du diéthylèneglycol, les éthers dialkyliques du triéthylèneglycol, les éthers dialkyliques du tétraéthylèneglycol, les éthers dialkyliques du pentaéthylèneglycol, les éthers dialkyliques de l'hexaéthylèneglycol, le DMSO et le THF.

7. Procédé selon la revendication 5 ou 6, **caractérisé en ce que** le fixateur de radicaux est choisi parmi les substances suivantes :
les dihydroxybenzènes, les trihydroxybenzènes, l'extrait de thé vert, l'extrait d'écorce de pin (pycnogénol), l'extrait de Ginkgo biloba (EGb 761), un mélange de flavonoïdes de plusieurs extraits de fruits et de légumes (GNLD), un bio-normalisateur (Sun-O Corp.), le DPPH (1,1-diphényl-2-picrylhydrazyle), le NDGA (acide nordihydroguaïarétique), le trolox (acide 6-hydroxy-2,5,7,8-tétraméthylchromanne-2-carboxylique), le 2,6-di-tert-butylphénol, le 4-méthyl-di-tert-butylphénol, le 4-méthoxy-di-tert-butylphénol, le 2,6-di-tert-butyl-p-crésol, l'acide 3,4-dihydroxybenzoïque, la vitamine C, la vitamine E, la vitamine Q, l'hydroquinone, l'ubiquinone, les lignanes, les hydroxyterpènes, les flavonoïdes, le curcumin, les tanins, les composés de l'acide rétinoïque, le Ge-132 sesquioxyde de bisbéta-carboxyéthylgermanium, la superoxyde-dismutase (SOD), la superoxyde-catalase, l'alpha-naphtoflavone, l'extrait de Ginkgo biloba (EGb 761), le dithionate de di(2-méthyl-5-chlorophényle) et les dérivés de Cu(II), les mébendazoles, l'extrait alcaloïde CS (soluble dans le chloroforme), la 4-(3,5-di-tert-butyl-4-hydroxyphényl)-3-hydroxy-1,2-naphtoquinone, la 4-(3,5-di-tert-butyl-4-hydroxyphényl)-3-méthoxy-1,2-naphtoquinone, la 4-(3, 5-di-tert-butyl-4-hydroxyphényl)-1,2-naphtoquinone, la 2-(3,5-di-tert-butyl-4-hydroxyphényl)-3-bromo-1,4-naphtoquinone, la 2-(3,5-di-tert-butyl-4-hydroxyphényl)-3-chloro-1,4-naphtoquinone, la 2-(3,5-di-tert-butyl-4-hydroxyphényl)-3-méthoxy-1,4-naphtoquinone, la 2-(3,5-di-tert-butyl-4-hydroxyphényl)-3-hydroxy-1,4-naphtoquinone, la 2-(3,5-di-tert-butyl-4-hydroxyphényl)-1,4-naphtoquinone, la 4-(3,5-di-tert-butyl-4-hydroxyphényl)-3-hydroxy-5,5,8,8-tétraméthyl-5,6,7,8-tétrahydro-1,2-anthraquinone, la 4-(3,5-di-tert-butyl-4-hydroxyphényl)-3-méthoxy-5,5,8,8-tétraméthyl-5,6,7,8-tétrahydro-1,2-anthraquinone, la 4-(3,5-di-tert-butyl-4-hydroxyphényl)-5,5,8,8-tétraméthyl-5,6,7,8-tétrahydro-1,2-anthraquinone, la 3-bromo-4-(3,5-di-tert-butyl-4-hydroxyphényl)-5,5,8,8-tétraméthyl-5,6,7,8-tétrahydro-1,2-anthraquinone, la 2-(3,5-di-tert-butyl-4-oxocyclohexa-2,5-diénylidène)-indane-1,3-dione, la 2-(3,5-di-tert-butyl-4-oxocyclohexa-2,5-diénylidène)-3,4-époxy-3-hydroxy-4-méthoxy-3,4-dihydro-2H-naphtalèn-1-one, la 2-(3,5-di-tert-butyl-4-oxocyclohexa-2,5-diénylidène)-3,4-époxy-3,4-diméthoxy-3,4-dihydro-2H-naphtalèn-1-one, la 2-(3,5-di-tert-butyl-4-hydroxyphényl)-indan-1-one, le 3,3-bi-[2-(3,5-di-tert-butyl-4-hydroxyphényl)-indèn-1-one]-3-yle, la 2-(3,5-di-tert-butyl-4-hydroxyphényl)-3-bromo-5,5,8,8-tétraméthyl-5,6, 7, 8-tétrahydro-1,4-anthraquinone, la 2-(3,5-di-tert-butyl-4-hydroxyphényl)-3-chloro-5,5,8,8-tétraméthyl-5,6,7,8-tétrahydro-1,4-anthraquinone, la 2-(3,5-di-tert-butyl-4-hydroxyphényl)-3-méthoxy-5,5,8,8-tétraméthyl-5,6,7,8-tétrahydro-1,4-anthraquinone, la 2-(3,5-di-tert-butyl-4-hydroxyphényl)-3-hydroxy-5,5,8,8-tétraméthyl-5,6,7,8-tétrahydro-1,4-anthraquinone, la 2-(3,5-di-tert-butyl-4-hydroxyphényl)-5,5,8,8-tétraméthyl-5,6,7,8-tétrahydro-1,4-anthraquinone, la 2-bromo-3- (3-bromo-5-tert-butyl-4-hydroxyphényl)-5,5,8,8-tétraméthyl-5,6,7,8-tétrahydro-1,4-anthraquinone, la 2-bromo-3-(3,5-dibromo-4-hydroxyphényl)-5,5,8,8-tétraméthyl-5,6,7,8-tétrahydro-1,4-anthraquinone, la 2-bromo-3- (3-bromo-5-tert-butyl-4-hydroxyphényl)-3-hydroxy-5,5,8,8-tétraméthyl-5,6,7,8-tétrahydro-1,4-anthraquinone, la 3-bromo-2-(3,5-di-tert-butyl-4-hydroxyphényl)-1,4-anthraquinone, la 2-(3,5-di-tert-butyl-4-hydroxyphényl)-3-méthoxy-1,4-anthraquinone, la 2-(3,5-di-tert-butyl-4-hydroxyphényl)-3-hydroxy-1,4-anthraquinone, le 5,5,8,8-tétraméthyl-5,6,7,8-tétrahydronaphtalène-1,3-diol, le 3-méthoxy-5,5,8,8-tétraméthyl-5,6,7,8-tétrahydronaphtalèn-1-ol, l'acide 4-(3-chloro-5,5,8,8-tétraméthyl-1,4-dioxo-1,4,5,6,7,8-hexahydroanthracèn-2-yl)-benzoïque, le 4-(3-chloro-5,5,8,8-tétraméthyl-1,4-dioxo-1,4,5,6,7,8-hexahydroanthracèn-2-yl)-benzoate de méthyle, l'acide 4-(3-hydroxy-1,4-dioxo-1,4-dihydronaphtalèn-2-yl)-benzoïque, l'acide (3-méthoxy-1,4-dioxo-1,4-dihydronaphtalèn-2-yl)-benzoate de méthyle, l'acide 4-(3-hydroxy-5,5,8,8-tétraméthyl-1,4-dioxo-1,4,5,6,7,8-hexahydroanthracèn-2-yl)-benzoïque, le 4-(3-hydroxy-1,4-dioxo-1,4-dihydronaphtalèn-2-yl-azo)-benzoate de méthyle, l'acide 4-(3-hydroxy-5,5,8,8-tétraméthyl-1,4-dioxo-1,4,5,6,7,8-hexahydroanthracèn-2-yl-azo)-benzoïque, la 3-(3,5-di-tert-butyl-4-oxocyclohexa-2,5-diénylidène)-5,5,8,8-tétraméthyl-5,6,7,8-tétrahydrocyclopenta[b]naphtalène-1,2-dione, la 3-(3,5-di-tert-butyl-4-oxocyclohexa-2,5-diénylidène)-5,5,8,8-tétraméthyl-5,6,7,8-tétrahydroanthracène-3H-1,2,4-trione, la 2-(3,5-di-tert-butyl-4-hydroxyphényl)-3-méthoxy-5,8-diméthyl-1,4-naphtoquinone, la 2-(3,5-di-tert-butyl-4-hydroxyphényl)-3-méthoxy-6,7-diméthyl-1,4-naphtoquinone, la 2-(3,5-di-tert-butyl-4-hydroxyphényl)-3-méthoxy-5-méthyl-1,4-naphtoquinone, la 2-(3, 5-di-tert-butyl-4-hydroxyphényl)-2-méthoxy-5-méthyl-1,4-naphtoquinone, la 2-(3,5-di-tert-butyl-4-hydroxyphényl)-3-méthoxy-6-méthyl-1,4-naphtoquinone, la 3-(3,5-di-tert-butyl-4-hydroxyphényl)-2-méthoxy-6-méthyl-1,4-naphtoquinone, la 2-(3,5-di-tert-butyl-4-hydroxyphényl)-3-méthoxy-5,6-diméthyl-1,4-naphtoquinone, la 3-(3, 5-di-tert-butyl-4-hydroxyphényl)-2-méthoxy-5,6-diméthyl-1, 4-naphtoquinone, la 2-(3,5-di-tert-butyl-4-hydroxyphényl)-3-méthoxy-5,7-diméthyl-1,4-naphtoquinone, la 3-(3, 5-di-tert-butyl-4-hydroxyphényl)-2-méthoxy-5,7-diméthyl-1,4-naphtoquinone, la 2-(3,5-di-tert-butyl-4-hydroxyphényl)-3-éthylthio-5-méthyl-1,4-naphtoquinone, la 2-(3,5-di-tert-butyl-4-hydroxyphényl)-3-éthylthio-6-méthyl-1,4-naphtoquinone, la 2-(3,5-di-tert-butyl-4-hydroxyphényl)-3-hydroxy-5,8-diméthyl-1,4-naphtoquinone, la 2-(3,5-di-tert-butyl-4-hydroxyphényl)-3-hydroxy-6,7-diméthyl-1,4-naphtoquinone, la 2-(3,5-di-tert-butyl-4-hydroxyphényl)-3-hydroxy-5-méthyl-1,4-naphtoquinone, la 3-(3,5-di-tert-butyl-4-hydroxyphényl)-2-hydroxy-5-méthyl-1,4-naphtoquinone, la 2-(3,5-di-tert-butyl-4-hydroxyphényl)-3-hydroxy-6-méthyl-1,4-naphtoquinone, la 3-(3,5-di-tert-butyl-4-hydroxyphényl)-2-hydroxy-6-méthyl-1,4-naphtoquinone, la 2-(3,5-di-tert-butyl-4-hydroxyphényl)-3-hydroxy-5,6-diméthyl-1,4-naphtoquinone, la 2-(3-bromo-5-tert-butyl-4-hydroxyphényl)-3-hydroxy-5,6-diméthyl-1,4-naphtoquinone, la 3-(3,5-di-tert-butyl-4-hydroxyphényl)-2-hydroxy-5,6-diméthyl-1,4-naphtoquinone, la 2-(3,5-di-tert-butyl-4-hydroxyphényl)-3-hydroxy-5,7-diméthyl-1,4-naphtoquinone, la 3-(3,5-di-tert-butyl-4-hydroxyphényl)-2-hydroxy-5,7-diméthyl-1,4-naphtoquinone.

8. Procédé selon l'une des revendications précédentes, **caractérisé en ce que** l'on sépare en totalité ou en partie des réactifs qui sont utilisés pour le traitement chimique de l'ADN, avant l'amplification effectuée ensuite.

9. Procédé selon l'une des revendications précédentes, **caractérisé en ce que** l'on dilue l'échantillon après le traitement chimique et avant l'amplification.

10. Procédé selon l'une des revendications précédentes, **caractérisé en ce que** l'on met en oeuvre simultanément dans un réacteur l'amplification de plusieurs segments d'ADN.

11. Procédé selon l'une des revendications précédentes, **caractérisé en ce que** l'on utilise pour l'amplification une ADN-polymérase résistante à la chaleur.

12. Procédé selon l'une des revendications précédentes, **caractérisé en ce que** l'on met en oeuvre une désulfonation de l'ADN avant les amplifications des revendications 1 ou 2.

13. Procédé selon l'une des revendications 1 à 12, **caractérisé en ce que** l'on détecte par des réactions d'hybridation les bases cytosines et/ou guanines restant après le deuxième traitement chimique.

14. Procédé selon l'une des revendications 1 à 12, **caractérisé en ce que** l'on détecte et/ou quantifie les bases cytosines et/ou guanines restant après le deuxième traitement chimique, par incorporation spécifique de marqueurs détectables sur les bases cytosines et/ou guanines.

15. Procédé selon l'une des revendications 1 à 12, **caractérisé en ce que**, dans l'étape d) selon la revendication 1 ou dans l'étape e) selon la revendication 2, on hybride, pour la détection de l'ADN prétraité, les produits d'amplification à une puce d'oligonucléotides, puis on procède aux étapes partielles suivantes :
a) on hybride l'ADN génomique amplifié à au moins un oligonucléotide, avec formation d'un duplex, les oligonucléotides hybridés en question délimitant , par leur extrémité 3', directement ou avec un écart allant jusqu'à 10 bases, les positions qui doivent faire l'objet d'un examen pour ce qui est de leur méthylation dans l'échantillon d'ADN génomique ;
b) on prolonge d'au moins un nucléotide l'oligonucléotide ayant une séquence connue de n nucléotides à l'aide d'une polymérase, le nucléotide portant un marqueur détectable et le prolongement dépendant de l'état de méthylation de la cytosine considérée dans l'échantillon d'ADN génomique.

16. Procédé selon l'une des revendications 1 à 12, **caractérisé en ce que** l'on détecte par des réactions de séquençage les bases cytosines et/ou guanines restant après le deuxième traitement chimique.

17. Procédé selon l'une des revendications 1 à 12, **caractérisé en ce que**, dans l'étape d) selon la revendication 1 ou dans l'étape e) selon la revendication 2, on hybride, pour la détection de l'ADN prétraité, les produits d'amplification à une puce d'oligonucléotides, puis on met en oeuvre les étapes partielles suivantes :
(a) on hybride un ensemble d'oligonucléotides à l'ADN génomique amplifié, avec formation d'un duplex, cet ensemble d'oligonucléotides étant constitué de deux espèces différentes et les oligonucléotides hybridés de la première espèce délimitant, par leur extrémité 3', directement ou avec un écart allant jusqu'à 10 bases, les positions qui doivent être examinées pour ce qui est de leur méthylation dans l'échantillon d'ADN génomique, et le deuxième oligonucléotide de la deuxième espèce s'hybridant à une deuxième région de la molécule cible, de telle sorte que l'extrémité 5' de l'oligonucléotide de la deuxième espèce soit séparée, par un espace ayant la taille d'un oligonucléotide unique, ou allant jusqu'à 10 nucléotides, de l'extrémité 3' de l'oligonucléotide hybridé de la première espèce, sur le site de la position sélectionnée en question ;
(b) on prolonge, à l'aide d'une polymérase, l'oligonucléotide de la première espèce, ayant une séquence connue de n nucléotides, d'au plus le nombre de nucléotides qui se trouvent entre l'extrémité 3' de l'oligonucléotide de la première espèce et l'extrémité 5' de l'oligonucléotide de la deuxième espèce, le prolongement dépendant de l'état de méthylation de la cytosine considérée dans l'échantillon d'ADN génomique
(c) on incube les oligonucléotides en présence d'une ligase, l'oligonucléotide de la première espèce prolongé par la réaction par polymérase et l'oligonucléotide de la deuxième espèce étant reliés l'un à l'autre, et on obtient de ce fait un produit de ligature, du moment que, dans l'étape précédente, un prolongement de l'oligonucléotide de la première espèce a eu lieu de telle sorte que l'extrémité 3', possédant une fonction 3'-hydroxy, de l'oligonucléotide prolongé est désormais directement délimitée par l'extrémité 5' de l'oligonucléotide de la deuxième espèce.

18. Procédé selon l'une des revendications 1 à 12, **caractérisé en ce que**, pour la détection de l'ADN prétraité selon l'étape d) de la revendication 1, on hybride les produits de la PCR sur une puce d'oligonucléotides, puis on met en oeuvre les étapes partielles suivantes :
(a) on hybride l'ADN génomique amplifié à au moins un oligonucléotide ayant une séquence connue de n nucléotides, avec formation d'un duplex, les oligonucléotides hybridés en question s'hybridant, par leur extrémité 3', en totalité ou en partie aux positions qui doivent être examinées pour ce qui est de leur méthylation dans l'échantillon d'ADN génomique ;
(b) on prolonge l'oligonucléotide, dans la mesure où il s'est hybridé au préalable, par son extrémité 3', sans défaut d'appariement de bases, à la position devant être étudiée, d'au moins un nucléotide et à l'aide d'une polymérase, au moins un nucléotide portant un_ marquage détectable et le prolongement dépendant de l'état de méthylation de la cytosine considérée de l'échantillon d'ADN génomique.

19. Procédé selon l'une des revendications précédentes, **caractérisé en ce que** l'on pourvoit d'un marqueur détectable les produits de la PCR et/ou les produits de prolongement et/ou les produits de ligature, pour permettre la détection.

20. Procédé selon l'une des revendications précédentes, **caractérisé en ce que** les marqueurs sont des marqueurs fluorescents.

21. Procédé selon l'une des revendications précédentes, **caractérisé en ce que** les marqueurs sont des radionucléides.

22. Procédé selon l'une des revendications 1 à 19, **caractérisé en ce que** les marqueurs sont des marqueurs de masse détachables, qui sont détectés dans un spectromètre de masse.

23. Procédé selon l'une des revendications précédentes, **caractérisé en ce que** l'une des amorces est liée à une phase solide lors de l'une des amplifications.

24. Procédé selon l'une des revendications 1 à 19, **caractérisé en ce que** l'on détecte globalement au spectromètre de masse les produits de la PCR et/ou les produits de prolongement et/ou les produits de ligature, et de ce fait on les caractérise sans ambiguïté par leur masse.

25. Procédé selon l'une des revendications 1 à 19, **caractérisé en ce que**, dans chaque cas, on détecte au spectromètre de masse un fragment des produits de la PCR et/ou des produits de prolongement et/ou des produits de ligature.

26. Procédé selon la revendication 25, **caractérisé en ce que** l'on produit par digestion avec une ou plusieurs exo- ou endonucléases le fragment du produit de la PCR et/ou du produit de prolongement et/ou du produit de ligature.

27. Procédé selon la revendication 25 ou 26, **caractérisé en ce que**, pour améliorer la détectabilité au spectromètre de masse, les fragments produits présentent une charge nette positive ou négative unique.

28. Procédé selon l'une des revendications 1 à 19, **caractérisé en ce que** l'on détecte et visualise les produits de la PCR et/ou les produits de prolongement et/ou les produits de ligature par une spectrométrie de masse à désorption/ionisation laser assistée par matrice (MALDI-TOF) ou par une spectrométrie de masse par électro-nébulisation (ESI).

29. Procédé selon l'une des revendications précédentes, dans lequel l'ADN génomique a été obtenu à partir d'un échantillon d'ADN, les sources d'ADN comprenant par exemple les lignées cellulaires, le sang, le crachat, les selles, l'urine, le liquide céphalo-rachidien, les tissus enrobés dans de la paraffine, par exemple les tissus des yeux, de l'intestin, des reins, du cerveau, du coeur, de la prostate, des poumons, du sein ou du foie, les porte-objets histologiques, et toutes leurs combinaisons possibles.

30. Utilisation d'un procédé selon l'une des revendications précédentes pour le diagnostic et/ou le pronostic d'événements indésirables pour des patients ou des individus, dans laquelle ces événements indésirables appartiennent à au moins l'une des catégories suivantes : les effets indésirables des médicaments; les maladies cancéreuses ; les dysfonctionnements, les lésions ou les maladies du SNC ; les symptômes d'agression ou les troubles du comportement ; les conséquences cliniques, psychologiques et sociales des lésions cérébrales ; les troubles psychotiques et les troubles de la personnalité ; la démence et/ou les syndromes associés ; les maladies, dysfonctionnements et lésions cardiovasculaires ; les dysfonctionnements, lésions ou maladies du tractus gastro-intestinal ; les dysfonctionnements, les lésions ou les maladies du système respiratoire ; les lésions, les inflammations, les infections, l'immunité et/ou la reconvalescence ; les dysfonctionnements, les lésions ou les maladies de l'organisme, en tant qu'anomalies du processus de développement ; les dysfonctionnements, les lésions ou les maladies de la peau, des muscles, du tissu conjonctif ou des os ; les dysfonctionnements, les lésions ou les maladies endocriniens et métaboliques ; les céphalées ou les dysfonctionnements sexuels.

31. Utilisation d'un procédé selon l'une des revendications 1 à 29 pour distinguer des types cellulaires ou des tissus, ou pour étudier la différenciation cellulaire.
